(19) Europäisches Patentamt / European Patent Office / Office européen des brevets

(11) **EP 3 533 777 B1**

(12) **EUROPEAN PATENT SPECIFICATION**

(45) Date of publication and mention
of the grant of the patent:
**26.07.2023 Bulletin 2023/30**

(21) Application number: **17863627.0**

(22) Date of filing: **24.02.2017**

(51) International Patent Classification (IPC):
**C07C 53/10** (2006.01)    **C07C 53/08** (2006.01)
**C07C 51/43** (2006.01)    **A61K 47/12** (2006.01)
**A61P 7/08** (2006.01)

(52) Cooperative Patent Classification (CPC):
(C-Sets available)
**A61P 7/08; C07C 51/43; C07C 53/08; C07C 53/10;**
C07B 2200/13                    (Cont.)

(86) International application number:
**PCT/JP2017/007248**

(87) International publication number:
**WO 2018/078901 (03.05.2018 Gazette 2018/18)**

(54) **HIGHER-ORDER ACETATE COMPOUND, AND SOLID DIALYSIS AGENT USING SAME**

ACETATVERBINDUNG HÖHERER ORDNUNG UND FESTER DIALYSEWIRKSTOFF MIT
VERWENDUNG DAVON

COMPOSÉ ACÉTATE D'ORDRE ÉLEVÉ, ET AGENT SOLIDE DE DIALYSE METTANT EN OUVRE
CELUI-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.10.2016  JP 2016212312**

(43) Date of publication of application:
**04.09.2019  Bulletin 2019/36**

(73) Proprietor: **Tomita Pharmaceutical Co., Ltd.
Naruto-shi, Tokushima 771-0360 (JP)**

(72) Inventors:
• **YOSHIMOTO, Yusuke
Naruto-shi
Tokushima 771-0360 (JP)**
• **KIKUISHI, Junya
Naruto-shi
Tokushima 771-0360 (JP)**

(74) Representative: **Mewburn Ellis LLP
Aurora Building
Counterslip
Bristol BS1 6BX (GB)**

(56) References cited:
WO-A1-2015/072494        CN-A- 104 892 397
JP-A- H0 782 209         JP-A- 2016 153 391
JP-A- 2016 216 447       US-A1- 2016 272 566

(52) Cooperative Patent Classification (CPC): (Cont.)

C-Sets
**C07C 51/43, C07C 53/08;**
**C07C 51/43, C07C 53/10**

**Description**

TECHNICAL FIELD

The present invention relates to a higher-order acetate compound containing acetic acid-sodium acetate mixed crystals. More specifically, the present invention relates to a higher-order acetate compound which contains acetic acid-sodium acetate mixed crystals, can reduce acetic acid odor, and further, can prevent decomposition of glucose even when coexisted with glucose. Furthermore, the present invention relates to a solid dialysis agent using the higher-order acetate compound.

BACKGROUND ART

In a hemodialysis solution, a bicarbonate dialysate using sodium bicarbonate for correcting acid-base equilibrium is mainstream, and it is also essential to blend acid to make the dialysate neutral. Also, when electrolyte components contained in a bicarbonate dialysate are circulated in coexistence in the same container, they generate carbon dioxide gas in the container and become extremely unstable, so that, as a dialysis agent used for preparation of dialysate, it is a common practice to produce two agents separately, an agent A mainly containing electrolyte components other than sodium bicarbonate and an agent B mainly containing sodium bicarbonate, and mix them at the time of use. Conventionally, as a dialysis agent A, there have been a liquid agent A containing electrolyte components in a concentrated liquid form and a solid agent A containing electrolyte components in a solid form, whereas the liquid agent A is regarded as a problem in terms of transportation cost, storage space in a hospital or the like, workability in a hospital, disposal of a container after use and the like, and recently, the solid dialysis agent A has become mainstream in Japan. Moreover, in recent years, there has been also proposed a three-pack type dialysis agent including an agent A mainly containing electrolyte components (other than sodium bicarbonate and sodium chloride), an agent S mainly containing sodium chloride, and an agent B mainly containing sodium bicarbonate, which makes it possible to change bicarbonate ion concentration and sodium ion concentration depending on a patient's condition during dialysis treatment (see Patent Document 1). Since acetic acid is metabolized in a short time and accumulation in a living body is small, in such dialysis agents, blending of acetic acid and sodium acetate as an acid and an alkaline agent for neutralizing the dialysate is widely performed.

In addition, acetic acid and sodium acetate are widely used not only in dialysis agents but also in various medicines, and further, generally used in the fields of cosmetics, foods and the like.

On the other hand, acetic acid has a disadvantage that it easily volatilizes and causes acetic acid odor. Therefore, in products containing acetic acid or sodium acetate, there is a problem of deteriorating environment at manufacturing sites and use sites due to generation of acetic acid odor. For example, in the field of dialysis treatment, dialysate is generally prepared at clinical sites by clinical engineering technicians. However, when preparing dialysate containing acetic acid, there is a problem that discomfort accompanied by irritating odor due to acetic acid odor occurs, workability deteriorates, and environment of clinical sites deteriorates.

Therefore, various methods for reducing acetic acid odor caused by volatilization of acetic acid have hitherto been proposed. For example, Patent Document 2 has disclosed that in a dialysis agent A, at least a part of acetic acid and acetates, including acetic acid and acetates, is an alkali metal salt of diacetate, and a molar ratio of acetic acid to acetates is set to 1 : 0.5 to 2, whereby acetic acid odor can be reduced in the dialysis agent A. In addition, Patent Document 3 has disclosed that in a dialysis agent A, volatilization of acetic acid can be prevented by making contained acetic acid substantially sodium diacetate. Furthermore, Patent Document 4 has reported that sodium diacetate crystals having a median diameter of 300 to 3000 $\mu$m and having a combination of specific diffraction peaks within a range of diffraction angle $2\Theta$ = 4.0 to 40.0° can prevent volatilization of acetic acid.
Patent Document 5 relates to a method for producing sodium diacetate using a gas phase reaction as the main reaction mode.

The technique of Patent Document 2 is a method for reducing acetic acid odor from a pharmaceutical point of view, and does not disclose an acetate compound with reduced acetic acid odor. Although the techniques of Patent Documents 3 and 4 attempt to reduce acetic acid odor by using an acetate compound having a specific structure, there is still room for improvement from the viewpoint of long-term storage property of a preparation.

On the other hand, glucose is contained in general dialysate to prevent hypoglycemia during dialysis treatment. Acetic acid also serves as a factor for decomposing glucose, so it is required to sufficiently consider stability of glucose in a solid dialysis agent using acetic acid and containing glucose. In the technique of Patent Document 2 described above, stability of glucose in the dialysis agent A can also be achieved by a pharmaceutical method. However, an acetate compound itself which can prevent decomposition of glucose has not been reported so far, regardless of the pharmaceutical method. Also, solid dialysis agents containing acetic acid also have a disadvantage such that solidification, coloration, pH fluctuation when dissolved in water and the like are caused by storage, resulting in poor preparation stability. Conventionally, there has been no report on an acetate compound having an effect of improving such preparation stability.

PRIOR ART DOCUMENTS

PATENT DOCUMENTS

Patent Document 1: Japanese Patent No. 5099464
Patent Document 2: Japanese Patent No. 5517321
Patent Document 3: Japanese Patent Laid-open Publication No. H7-59846
Patent Document 4: WO 2015/72494
Patent Document 5: CN 104 892 397 A

SUMMARY OF THE INVENTION

PROBLEMS TO BE SOLVED BY THE INVENTION

An object of the present invention is to provide a higher-order acetate compound which can reduce acetic acid odor, and further, can prevent decomposition of glucose even when coexisted with glucose. Still another object of the present invention is to provide a solid dialysis agent using the higher-order acetate compound.

MEANS FOR SOLVING THE PROBLEM

The present inventors have conducted intensive studies to solve the above problems, and found that a higher-order acetate compound according to claim 1 can effectively prevent volatilization of acetic acid, can reduce acetic acid odor, and also can prevent decomposition of glucose even when coexisted with glucose. Further, the present inventors have found that, in addition to the reduction of acetic acid odor and the prevention of decomposition of glucose, a solid dialysis agent containing the higher-order acetate compound prevents solidification, coloration, pH fluctuation when dissolved in water and the like due to storage, and has excellent preparation stability. The present invention has been accomplished by further studies based on such knowledge.
That is, the present invention provides inventions of the following aspects. In one aspect, the present invention relates to a higher-order acetate compound according to claim 1. In a further aspect, the present invention relates to a solid dialysis agent A according to claim 2.
In a further aspect, the present invention relates to a solid dialysis agent according to claim 4. The solid dialysis agent may be a two-pack type dialysis agent according to claim 5 or a three-pack bicarbonate dialysis agent according to claim 6.
In a further aspect, the present invention relates to a method for producing the higher order acetate compound according to claim 7.

ADVANTAGES OF THE INVENTION

Since the higher-order acetate compound of the present invention can effectively prevent volatilization of acetic acid and can reduce acetic acid odor, it can prevent occurrence of an unpleasant odor due to volatilization of acetic acid of products or at manufacturing sites, in various fields of medicines, foods, cosmetics, and the like. In addition, since the higher-order acetate compound of the present invention can prevent decomposition of glucose even when it coexists with glucose, it can be suitably used as an additive to a product containing glucose.
Further, in addition to the reduction of acetic acid odor and the prevention of decomposition of glucose, a solid dialysis agent containing the higher-order acetate compound of the present invention can prevent solidification, coloration, pH fluctuation when dissolved in water and the like due to storage, so that it can have excellent preparation stability.

BRIEF DESCRIPTION OF THE DRAWINGS

Fig. 1 is a diagram showing a powder X-ray diffraction pattern of a higher-order acetate compound obtained in Example 1.
Fig. 2 is a diagram showing a powder X-ray diffraction pattern of a higher-order acetate compound obtained in Example 2.

Fig. 3 is a diagram showing a powder X-ray diffraction pattern of a higher-order acetate compound obtained in Example 3.

Fig. 4 is a diagram showing a powder X-ray diffraction pattern of a higher-order acetate compound obtained in Example 4.

Fig. 5 is a diagram showing a powder X-ray diffraction pattern of a higher-order acetate compound obtained in Example 5.

Fig. 6 is a diagram showing a powder X-ray diffraction pattern of a higher-order acetate compound obtained in Example 6.

Fig. 7 is a diagram showing a powder X-ray diffraction pattern of a higher-order acetate compound obtained in Example 7.

Fig. 8 is a diagram showing a powder X-ray diffraction pattern of a higher-order acetate compound obtained in Example 8.

Fig. 9 is a diagram showing a powder X-ray diffraction pattern of a higher-order acetate compound obtained in Example 9.

Fig. 10 is a diagram showing a powder X-ray diffraction pattern of a higher-order acetate compound obtained in Example 10.

Fig. 11 is a diagram showing a powder X-ray diffraction pattern of a higher-order acetate compound obtained in Comparative Example 1.

Fig. 12 is a diagram showing a powder X-ray diffraction pattern of a higher-order acetate compound obtained in Comparative Example 2.

Fig. 13 is a photograph obtained by observing a crystal form of the higher-order acetate compound obtained in Example 2 with a scanning electron microscope.

Fig. 14 is a diagram showing a powder X-ray diffraction pattern of anhydrous sodium acetate.

## EMBODIMENTS OF THE INVENTION

### 1. Higher-order acetate compound

The higher-order acetate compound of the present invention is a higher-order acetate compound containing acetic acid-sodium acetate mixed crystals, characterized in that a diffraction peak A at $2\Theta = 8.8° \pm 0.2°$ and a diffraction peak B at $2\theta = 22.3° \pm 0.2°$ are observed in powder X-ray diffraction measurement, and a ratio Ia/Ib of integrated intensity Ia of the diffraction peak A to integrated intensity Ib of the diffraction peak B is 0.001 to 1.140. Hereinafter, the higher-order acetate compound of the present invention will be described in detail.

[Composition]

The higher-order acetate compound of the present invention is a higher-order acetate compound containing acetic acid-sodium acetate mixed crystals. In the present invention, the "acetic acid-sodium acetate mixed crystals" refer to crystals formed by mixing acetic acid and sodium acetate with each other. Further, in the present invention, the "higher-order acetate compound" refers to a compound formed by combining acetic acid (primary compound) and sodium acetate (primary compound) with each other. That is, in the present invention, the "higher-order acetate compound containing acetic acid-sodium acetate mixed crystals" is a compound formed by combining acetic acid and sodium acetate with each other, which contains crystals formed by mixing these substances. The "higher-order acetate compound containing acetic acid-sodium acetate mixed crystals" in the present invention may contain components other than water, acetic acid and sodium acetate in amounts not to adversely affect the effect of the present invention.

The molar ratio of acetic acid to sodium acetate constituting the higher-order acetate compound of the present invention is not particularly limited as long as it has a powder X-ray diffraction pattern to be described later, and specifically, the molar ratio of acetic acid to sodium acetate is usually 1 : 0.5 to 10, preferably 1 : 0.5 to 3.0, and further preferably 1 : 0.7 to 2.0. Even though the molar ratio of acetic acid among constituents is high as described above, the higher-order acetate compound of the present invention can overcome the disadvantages of acetic acid such as acetic acid odor, decomposition of glucose, and destabilization of preparation of solid dialysis agent (solidification, coloration, and pH fluctuation when dissolved in water).

[Characteristics of powder X-ray diffraction pattern]

In the higher-order acetate compound of the present invention, a diffraction peak A at $2\Theta = 8.8° \pm 0.2°$ and a diffraction peak B at $2\Theta = 22.3° \pm 0.2°$ are observed in powder X-ray diffraction measurement. In the higher-order acetate compound of the present invention, a ratio Ia/Ib of integrated intensity Ia of the diffraction peak A to integrated intensity Ib of the diffraction peak B is 0.001 to 1.140. In the higher-order acetate compound of the present invention, since the diffraction peak A is observed, the ratio Ia/Ib is a value greater than 0. By satisfying such a ratio, it is possible to effectively prevent volatilization of acetic acid to reduce acetic acid odor, and it is also possible to have an effect of preventing decomposition of glucose even when coexisted with glucose. The ratio Ia/Ib in the higher-order acetate compound of the present invention is further preferably 0.006 to 1.440, and particularly preferably 0.060 to 0.760, from the viewpoint of further improving the effect of preventing volatilization of acetic acid and the effect of preventing decomposition of glucose.

Also, as one of the preferred characteristics of the powder X-ray diffraction pattern of the higher-order acetate compound of the present invention, in addition to the above peaks A and B, a peak at $17.0° \pm 0.2°$ (diffraction peak C) is observed within a scanning range of $2\theta = 5$ to $50°$, in the powder X-ray diffraction measurement. Furthermore, one preferable aspect of the higher-order acetate compound of the present invention includes those having a ratio Ia/Ic of the integrated intensity Ia to an integrated intensity Ic of the diffraction peak C satisfying 1.000 to 45.000 or those satisfying 1.000 to 350.000, from the viewpoint of further improving the effect of preventing volatilization of acetic acid and the effect of preventing decomposition of glucose.

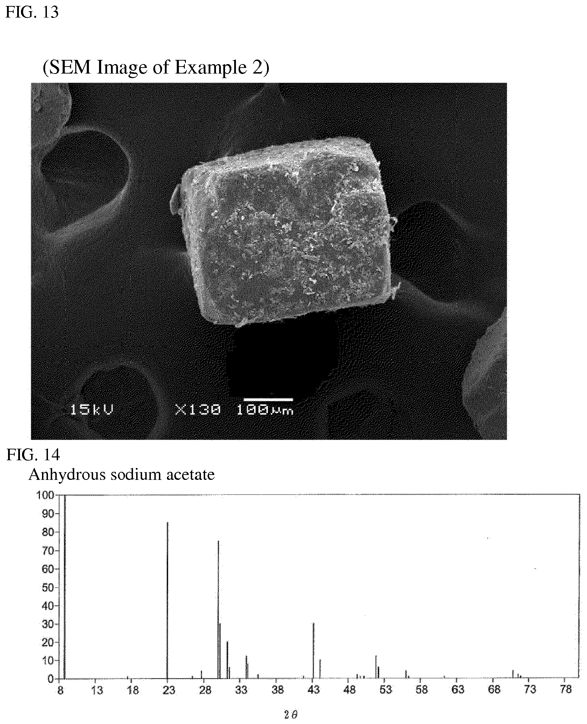

Further, another characteristic of the powder X-ray diffraction pattern of the higher-order acetate compound of the present invention includes a combination of peaks of $11.1° \pm 0.2°$, $13.6° \pm 0.2°$, $15.8° \pm 0.2°$, $17.0° \pm 0.2°$, $19.2° \pm 0.2°$, $20.9° \pm 0.2°$, $23.8° \pm 0.2°$, $25.1° \pm 0.2°$, $30.8° \pm 0.2°$, $34.8° \pm 0.2°$, $36.6° \pm 0.2°$, $38.4° \pm 0.2°$, $40.9° \pm 0.2°$, $42.5° \pm 0.2°$, $45.6° \pm 0.2°$, $46.4° \pm 0.2°$ and $47.1° \pm 0.2$, within the scanning range of $2\Theta = 5$ to $50°$, in the powder X-ray diffraction measurement. These peaks are peaks not observed with anhydrous sodium acetate. The powder X-ray diffraction pattern of anhydrous sodium acetate is as shown in Fig. 14.

Further, one aspect of the powder X-ray diffraction pattern of the higher-order acetate compound of the present invention includes, in addition to the peaks A and B, $11.1° \pm 0.2°$, $13.6° \pm 0.2°$, $15.8° \pm 0.2°$, $17.0° \pm 0.2°$, $19.2° \pm 0.2°$, $20.9° \pm 0.2°$, $23.8° \pm 0.2°$, $25.1° \pm 0.2°$, $26.3° \pm 0.2°$, $30.8° \pm 0.2°$, $33.8° \pm 0.2°$, $34.8° \pm 0.2°$, $35.7° \pm 0.2°$, $36.6° \pm 0.2°$, $38.4° \pm 0.2°$, $40.9° \pm 0.2°$, $41.7° \pm 0.2°$, $42.5° \pm 0.2°$, $45.6° \pm 0.2°$, $46.4° \pm 0.2°$, $47.1° \pm 0.2°$ and $49.2° \pm 0.2°$, within the scanning range of $2\theta = 5$ to $50°$, in the powder X-ray diffraction measurement.

The powder X-ray diffraction pattern can be determined by a powder X-ray diffraction method under the following conditions.

Target: Cu
X-ray tube current: 30 mA
X-ray tube voltage: 40 kV
Scanning range: $2\Theta = 5$ to $90°$
Pretreatment of measurement sample: ground in agate mortar

[Particle size]

The particle size of the higher-order acetate compound of the present invention may vary depending on production conditions and the like, and is generally 50 to 1500 $\mu$m, preferably 100 to 1000 $\mu$m, and further preferably 150 to 800 $\mu$m, as the median diameter (D50). In the present invention, the median diameter of the higher-order acetate compound is a particle size of 50% weight cumulative calculated from the result measured according to "General Tests, Processes and Apparatus, 3.04 Particle Size Determination, 2. Method 2. Analytical Sieving Method" described in The Japanese Pharmacopoeia, Seventeenth Edition, using a 75 mm JIS standard sieve.

The median diameter (D50) is calculated from the result of the particle size distribution measured by the sieving method according to the following formula.

[Expression 1]

$$y(50) = \left[ \left\{ \frac{(y_2 - y_1) \times 100}{(x_2 - x_1)} \right\} (50 - x_1) \right] / 100 + y_1$$

y(50): median diameter
y1, y2: sieve opening ($\mu$m) before and after weight cumulative distribution is 50%
x1, x2: cumulative distribution (%) on the sieve before and after weight cumulative distribution is 50%

[Use]

The higher-order acetate compound of the present invention can be used in the fields of medicines, foods, cosmetics and the like, as a pH adjuster, a supply source of acetic acid, a supply source of sodium, and the like. In particular, since the higher-order acetate compound of the present invention can prevent decomposition of glucose in the coexistence of glucose, it can be suitably used for various solid compositions containing glucose.

Since a solid dialysis agent is dissolved in water to prepare dialysate at clinical sites, it is a preparation strongly requiring reduction of acetic acid odor. Also, the solid dialysis agent is often blended with glucose, and is a preparation also requiring maintenance of glucose stability. Since the higher-order acetate compound of the present invention can satisfy such required characteristics of the solid dialysis agent, it can be particularly suitably used as an additive to be blended in the solid dialysis agent. In addition, the solid dialysis agent containing the higher-order acetate compound of the present invention can prevent solidification, coloration and pH fluctuation after dissolution in water due to storage, and can also improve preparation stability.

Specific aspects of the solid dialysis agent containing the higher-order acetate compound of the present invention will be described later.

[Production method]

The method for producing the higher-order acetate compound of the present invention is not particularly limited as long as it has the above-mentioned powder X-ray diffraction pattern and a higher-order acetate compound containing acetic acid-sodium acetate mixed crystals is obtained, and preferred examples include a production method including the following steps 1 and 2.

Step 1: a step of mixing acetic acid, sodium acetate and an aqueous solvent to obtain a mixed solution

Step 2: a step of drying the mixed solution obtained in the step 1 under reduced pressure, until a higher-order acetate compound is produced, in which the diffraction peak A at $2\theta = 8.8° \pm 0.2°$ and a diffraction peak B at $2\theta = 22.3° \pm 0.2°$ are observed and a ratio Ia/Ib of integrated intensity Ia of the diffraction peak A to integrated intensity of Ib of the diffraction peak B is from 0.001 to 1.140.

The steps 1 and 2 will be described below.

(Step 1)

In step 1, acetic acid, sodium acetate and an aqueous solvent are used as raw materials to obtain a mixed solution.

The acetic acid used in step 1 may be acetic acid which is generally used in the fields of pharmaceuticals, foods, cosmetics and the like, and preferred examples include glacial acetic acid.

Also, the sodium acetate used in step 1 may be any one generally used in the fields of pharmaceuticals, foods, cosmetics and the like, and may be either a hydrous or anhydrous state, and preferred examples include anhydrous sodium acetate.

The aqueous solvent used in step 1 is water or a mixed solvent of water and an organic solvent having compatibility with water.

When the aqueous solvent is a mixed solvent, the type of the organic solvent used in the mixed solvent is not particularly limited as long as it has compatibility with water (solubility in water), can dissolve acetic acid and sodium acetate, and can be distilled by drying under reduced pressure in step 2 to be described later, and examples include lower alcohols such as methanol, ethanol, propanol, and butanol. In the mixed solvent, the organic solvents may be contained alone or in combination of two or more kinds thereof.

As the aqueous solvent used in step 1, water is preferably used.

In addition, in step 1, the ratio of acetic acid and sodium acetate to be mixed as raw materials is the same as the ratio shown in the section of [Composition].

Also, in step 1, the ratio of the aqueous solvent to be used to the total amount of acetic acid and sodium acetate is not particularly limited, and for example, the ratio is about 5 to 300 parts by weight of the aqueous solvent to 100 parts by weight of the total amount of acetic acid and sodium acetate. From the viewpoint of efficiently producing the higher-order acetate compound of the present invention, the ratio of the aqueous solvent to 100 parts by weight of the total amount of acetic acid and sodium acetate is preferably 5 to 100 parts by weight, and further preferably 10 to 50 parts by weight.

In step 1, a mixed solution is obtained by mixing acetic acid, sodium acetate and an aqueous solvent, and it is preferred that the mixed solution is in a state where acetic acid and sodium acetate are dissolved in an aqueous solvent, from the viewpoint of efficiently producing the higher-order acetate compound of the present invention.

In order to obtain a mixed solution in a state where acetic acid and sodium acetate are dissolved in an aqueous solvent, a mixture of acetic acid, sodium acetate and an aqueous solvent should be subjected to heat treatment. The temperature condition of the heat treatment is not particularly limited, and is, for example, 20 to 200°C, preferably 30 to 150°C, and further preferably 40 to 125°C. Regarding heating time of the heat treatment, conditions under which acetic acid and sodium acetate can be dissolved in an aqueous solvent may be appropriately set according to production scale, temperature conditions and the like employed, for example, heating time in a state where the temperature has been reached is about 1 to 60 minutes, preferably about 10 to 50 minutes, and further preferably about 20 to 30 minutes.

Moreover, in the heat treatment, a part of the aqueous solvent may be volatilized so that acetic acid and sodium acetate in the mixed solution are concentrated. Further, in the heat treatment, stirring may be performed, or bubbling may be performed by supplying air.

The mixed solution thus obtained is subjected to step 2 to be described later. When heating is performed during the preparation of the mixed solution, it should be cooled down to about 10 to 70°C if necessary, and subjected to step 2 described later.

(Step 2)

In step 2, the mixed solution obtained in the step 1 is dried under reduced pressure, until a higher-order acetate compound in which the peaks A and B are observed and a ratio Ia/Ib satisfies the above range is produced. In this manner, the mixed solution obtained in the step 1 is dried under reduced pressure, whereby mixed crystals of acetic acid-sodium acetate are precipitated, and the higher-order acetate compound of the present invention can be produced.

In step 2, the mixed solution obtained in the step 1 may be subjected to drying under reduced pressure as it is, and if necessary, the mixed solution obtained in the step 1 may be added with a seed crystal composed of the higher-order acetate compound of the present invention, and then subjected to drying under reduced pressure.

The pressure condition of the drying under reduced pressure in step 2 may be appropriately set according to the temperature condition, the amount of the mixed solution and the like, and is usually -30 to -100 kPa. From the viewpoint of efficiently producing the higher-order acetate compound of the present invention, the pressure condition of the drying under reduced pressure is preferably -40 to -100 kPa, and further preferably -50 to -100 kPa.

The temperature condition of the drying under reduced pressure in step 2 may be appropriately set according to production scale, pressure condition, the amount of the mixed solution and the like employed, and is usually 30 to 190°C or 30 to 150°C.

More specifically, as a preferred example of the temperature condition of the drying under reduced pressure in step 2, from the viewpoint of efficiently producing the higher-order acetate compound of the present invention, it is more preferably 35 to 120°C, and further preferably 40 to 110°C. In particular, as a more preferred example of the temperature condition of the drying under reduced pressure in step 2, it is more preferably 40 to 100°C, and particularly preferably 50 to 95°C, and an aspect in which the temperature condition is operated so as to raise the heating temperature in stages, at the same speed as the boiling point increase occurring with progress of the drying under reduced pressure.

Also, as another preferable example of the temperature condition of the drying under reduced pressure in step 2, from the viewpoint of efficiently producing the higher-order acetate compound of the present invention, it is more preferably 50 to 190°C, and further preferably 50 to 150°C. In particular, as another more preferred example of the temperature condition of the drying under reduced pressure in step 2, it is more preferably 55°C to 145°C, and particularly preferably 60°C to 135°C, and an aspect in which the temperature condition is operated so as to raise the heating temperature in stages, at the same speed as the boiling point increase occurring with progress of the drying under reduced pressure.

Further, in the drying under reduced pressure in step 2, outside air, clean air, or dry air may be ventilated if necessary, within a range that satisfies the pressure condition described above. The ventilation method is not particularly limited, and examples thereof include a method of bubbling in the mixed solution during the drying under reduced pressure, a method of ventilating to bring air into contact with a liquid surface of the mixed solution, and the like.

In the case of ventilation during the drying under reduced pressure, there are no particular restrictions on an air discharge site, and examples thereof include a bottom of a can body, a stirring blade, a wall of a can body, and the like. Drying is accelerated by ventilation, which makes production in a shorter time possible. When bubbling is performed during the drying under reduced pressure, ventilation is started before introduction of raw materials in order to prevent intrusion of contents into ventilation openings, and when the same device is used in steps 1 and 2 and, if necessary, after step 2, it is desirable not to stop ventilation. The amount of gas to be ventilated may be appropriately set according to production scale, pressure condition, the amount of the mixed solution and the like employed, and is usually 5 to 300 L/min, preferably 10 to 150 L/min, and further preferably 15 to 100 L/min at an atmospheric pressure of 1 atm at 25°C per 100 kg of raw materials.

The mixed solution obtained in the above step 1 is dried under reduced pressure to precipitate a higher-order acetate compound containing mixed crystals of acetic acid-sodium acetate. However, when the time of drying under reduced pressure is short, peak A is not observed in the precipitated higher-order acetate compound, and the higher-order acetate compound of the present invention cannot be obtained. On the other hand, when crystals of the higher-order acetate compound in which peak A is not observed by drying under reduced pressure is precipitated and then drying under reduced pressure is subsequently continued, the peaks A and B are observed in the precipitated higher-order acetate compound, and crystals of a higher-order acetate compound in which a ratio Ia/Ib satisfies the above range are produced. Therefore, with respect to the time of drying under reduced pressure in step 2, the conditions are set such that crystals of a higher-order acetate compound in which the peaks A and B are observed and a ratio Ia/Ib satisfies the above range are precipitated. Specifically, the time of drying under reduced pressure in step 2 is appropriately set so that crystals of the higher-order acetate compound of the present invention are precipitated according to the pressure condition, the temperature condition, the liquid amount of the mixed solution and the like, and drying under reduced pressure should be terminated, for example, when the product temperature (the temperature of the precipitated higher-order acetate compound) reaches 45°C to 95°C, and preferably 60°C to 90°C, with the above-mentioned conditions satisfied. In addition, the time of drying under reduced pressure until reaching such product temperature is, for example, 0.5 to 24 hours, preferably 1 to 15 hours, and further preferably 2 to 9 hours or 1 to 3 hours.

The higher-order acetate compound of the present invention precipitated in step 2 should be recovered after cooling to around room temperature, if necessary. Also, the higher-order acetate compound precipitated in step 2 may be further subjected to a drying treatment such as a shelf dryer or a fluidized bed dryer, if necessary.

In addition, if necessary, the particle size of the recovered higher-order acetate compound of the present invention may be adjusted using a sieve or the like.

Moreover, although steps 1 and 2 may be carried out by different devices, steps 1 and 2 may be carried out in one pot using a device including mixing means, heating means, and reduced pressure drying means.

2. Solid dialysis agent

The "solid dialysis agent" refers to a solid raw material used for preparing dialysate. The solid dialysis agent of the present invention contains the higher-order acetate compound. Since the acetic acid odor of the higher-order acetate compound is reduced, deterioration of the working environment at the time of preparation of dialysate at clinical sites can be prevented. In addition, the higher-order acetate compound can also prevent decomposition of glucose, so that when the solid dialysis agent of the present invention contains glucose, glucose can also be stabilized. Furthermore, since the solid dialysis agent of the present invention can prevent solidification, coloration and pH fluctuation after dissolution in water caused by storage, by containing the higher-order acetate compound, it can also have excellent preparation stability.

[Type of solid dialysis agent]

The solid dialysis agent of the present invention may be used for preparation of either a hemodialysis solution or a peritoneal dialysis solution, and is preferably used for preparation of a hemodialysis solution. In particular, the solid dialysis agent of the present invention is suitable as a dialysis agent used for preparation of a bicarbonate dialysate containing bicarbonate ions, that is, a bicarbonate dialysis agent.

[Specific aspect in case of being used as bicarbonate dialysis agent]

(Composition of bicarbonate dialysis agent)

When the solid dialysis agent of the present invention is used as a bicarbonate dialysis agent, the higher-order acetate compound as a supply source of acetate ions and sodium bicarbonate as a supply source of bicarbonate ions are contained, and other physiologically available electrolytes used for dialysate are further contained. Examples of such electrolytes include those which can serve as supply sources of magnesium ions, calcium ions, sodium ions, potassium ions, chloride ions, citrate ions, lactate ions, gluconate ions, succinate ions, malate ions, and the like. Among them, one serving as at least a supply source of sodium ions, chloride ions, magnesium ions and calcium ions is preferably contained, and in addition to them, one serving as a supply source of potassium ions is further preferably contained.

Examples of the component serving as a supply source of magnesium ions include magnesium salts. The magnesium salt to be used in the dialysis agent of the present invention is not particularly limited as long as it is acceptable as a component of the dialysate, and examples thereof include magnesium chloride, magnesium lactate, magnesium citrate, magnesium gluconate, magnesium succinate, magnesium malate, and the like. Among these magnesium salts, magnesium chloride has high solubility in water, and is thus suitably used as a supply source of magnesium. These magnesium salts may be in the form of hydrates. Also, one of these magnesium salts may be used alone or in combination of two or more kinds.

Examples of the component serving as a supply source of calcium ions include calcium salts. The calcium salt to be used in the dialysis agent of the present invention is not particularly limited as long as it is acceptable as a component of the dialysate, and examples thereof include calcium chloride, calcium lactate, calcium citrate, calcium gluconate, calcium succinate, calcium malate, and the like. Among these calcium salts, calcium chloride has high solubility in water, and is thus suitably used as a supply source of calcium. These calcium salts may be in the form of hydrates. Also, one of these calcium salts may be used alone or in combination of two or more kinds.

Examples of the component serving as a supply source of sodium ions include sodium salts. The higher-order acetate compound also serves as a supply source of sodium ions, but by using a sodium salt other than the higher-order acetate compound, it is possible to supplement sodium ions to provide the dialysate with a desired sodium ion concentration. The sodium salt is not particularly limited as long as it is acceptable as a component of the dialysate, and examples thereof include sodium chloride, sodium lactate, sodium citrate, sodium gluconate, sodium succinate, sodium malate, and the like. Among these sodium salts, sodium chloride is suitably used as a supply source of sodium since it is the most physiological substance. These sodium salts may be in the form of hydrates. Also, one of these sodium salts may be used alone or in combination of two or more kinds.

Examples of the component serving as a supply source of potassium ions include potassium salts. The potassium salt to be blended in the dialysis agent of the present invention is also not particularly limited as long as it is acceptable as a component of the dialysate, and examples thereof include potassium chloride, potassium lactate, potassium citrate, potassium gluconate, potassium succinate, potassium malate, and the like. Among these potassium salts, potassium chloride is suitably used as a supply source of potassium since chloride ion is the most physiological substance. These potassium salts may be in the form of hydrates. Also, one of these potassium salts may be used alone or in combination of two or more kinds.

Examples of the component serving as a supply source of chloride ions include chloride salts. The chloride salt to be blended in the dialysis agent of the present invention is also not particularly limited as long as it is acceptable as a component of the dialysate, and examples thereof include sodium chloride, calcium chloride, magnesium chloride, potassium chloride, and the like. These chloride salts are suitably used because they have high solubility in water and can also serve as a supply source of sodium, potassium, magnesium, or potassium. These chloride salts may be in the form of hydrates. Also, one of these chloride salts may be used alone or in combination of two or more kinds. In addition, hydrochloric acid which also serves as a pH adjusting agent can also be used as a supply source of chloride ions.

When the solid dialysis agent of the present invention is used as a bicarbonate dialysis agent, the type and combination of the electrolytes to be blended in addition to the higher-order acetate compound and sodium bicarbonate are appropriately set according to a composition of each ion contained in the finally prepared dialysate, and examples of the electrolyte (other than the above higher-order acetate compound and sodium bicarbonate) contained in the solid dialysis agent include sodium chloride, magnesium chloride, chloride calcium, and potassium chloride. In addition, when sodium chloride, magnesium chloride, calcium chloride, and potassium chloride are used in combination as electrolytes, an organic acid salt (other than acetate) may be further contained. Examples of such an organic acid salt include sodium lactate, sodium gluconate, sodium citrate, sodium malate, sodium succinate and the like. These organic acid salts may be used alone or in combination of two or more kinds.

Moreover, when the solid dialysis agent of the present invention is used as a bicarbonate dialysis agent, glucose may be contained to prevent hypoglycemia during dialysis treatment. Even when the higher-order acetate compound coexists with glucose, it can prevent decomposition of glucose. Therefore, when glucose is contained, it can be blended in the same preparation as the higher-order acetate compound.

A bicarbonate dialysate containing acetate ions is usually formulated to have the composition and concentration shown in Table 1 below. Accordingly, when the solid dialysis agent of the present invention is used as a bicarbonate dialysis agent, the higher-order acetate compound, sodium bicarbonate, and other components (other electrolyte components and glucose) should be blended so that the composition and concentration of the bicarbonate dialysate finally prepared satisfy the following ranges.

[Table 1]

| Ions and components contained in bicarbonate dialysate | Concentration in bicarbonate dialysate |
| --- | --- |
| Acetate ions | 2 to 12 mEq/L, preferably 3 to 10 mEq/L |
| Bicarbonate ions | 20 to 40 mEq/L, preferably 25 to 35 mEq/L |
| Sodium ions | 120 to 150 mEq/L, preferably 135 to 145 mEq/L |
| Potassium ions | 0 to 3 mEq/L, preferably 1.5 to 2.5 mEq/L |
| Calcium ions | 1.5 to 4.5 mEq/L, preferably 2.0 to 3.5 mEq/L |

(continued)

| Ions and components contained in bicarbonate dialysate | Concentration in bicarbonate dialysate |
|---|---|
| Magnesium ions | 0 to 2.0 mEq/L, preferably 0.5 to 1.5 mEq/L |
| Citrate ions | 0 to 18 mEq/L, preferably 0 to 3 mEq/L |
| Chloride ions | 90 to 135 mEq/L, preferably 100 to 120 mEq/L |
| Lactate ions | 0 to 10 mEq/L |
| Malate ions | 0 to 10 mEq/L |
| Gluconate ions | 0 to 10 mEq/L |
| Succinate ions | 0 to 10 mEq/L |
| Glucose | 0 to 2.5 g/L, preferably 1.0 to 1.5 g/L |

For example, in the solid dialysis agent of the present invention, when magnesium chloride, calcium chloride and potassium chloride are used as the electrolytes, in addition to the higher-order acetate compound and sodium bicarbonate, in order that the concentrations of each ion contained in the dialysate satisfy the ranges shown in Table 1, the ratio of magnesium chloride, calcium chloride, and potassium chloride in the dialysis agent should be set to 2 to 12 mol and preferably 3 to 10 mol by the total number of moles of acetic acid and sodium acetate constituting the higher-order acetate compound, 0.75 to 2.25 mol and preferably 1.0 to 1.75 mol of calcium chloride, 0 to 3 mol and preferably 1.5 to 2.5 mol of potassium chloride, and 20 to 40 mol and preferably 25 to 35 mol of bicarbonate sodium, based on 0.5 mol of magnesium chloride.

The solid dialysis agent of the present invention can be adjusted to have a pH of the bicarbonate dialysate in a reasonable range depending on the higher-order acetate compound, and further, may separately contain a pH adjusting agent, if necessary. The pH adjusting agent that can be used for the dialysis agent of the present invention is not particularly limited as long as it is acceptable as a component of the dialysate, and examples thereof include liquid acids such as hydrochloric acid, lactic acid and gluconic acid, solid acids such as citric acid, succinic acid, fumaric acid, malic acid and glucono delta-lactone, sodium, potassium, calcium and magnesium salts thereof, and the like. Among these pH adjusting agents, organic acids are suitably used. The pH adjusting agents may be used alone or in combination of two or more kinds.

(Type of bicarbonate dialysis agent)

As bicarbonate dialysis agents, there are known one-pack type in which all components contained in a bicarbonate dialysate are mixed in one preparation; two-pack type including a dialysis agent A containing electrolyte components other than sodium bicarbonate and, if necessary, glucose, and a dialysis agent B containing sodium bicarbonate; three-pack type including a dialysis agent A-1 containing electrolyte components other than sodium bicarbonate, a dialysis agent A-2 containing glucose, and a dialysis agent B containing sodium bicarbonate; and three-pack type including a dialysis agent S containing sodium chloride, a dialysis agent B containing sodium bicarbonate, and a dialysis agent A containing electrolyte components other than sodium chloride and sodium bicarbonate and, if necessary, glucose. When the solid dialysis agent of the present invention is used as a bicarbonate dialysis agent, any of these types of bicarbonate dialysis agents may be used.

Where the solid dialysis agent of the present invention is a one-pack type bicarbonate dialysis agent, the bicarbonate dialysis agent should be formulated by using the higher-order acetate compound, sodium bicarbonate, other electrolytes, and, if necessary, glucose.

When the solid dialysis agent of the present invention is a two-pack type bicarbonate dialysis agent including a dialysis agent A and a dialysis agent B, the dialysis agent A should be formulated by using the higher-order acetate compound, other electrolytes (other than the higher-order acetate compound and sodium bicarbonate), and, if necessary, glucose. Also, the dialysis agent B of the two-pack type bicarbonate dialysis agent may be formulated by using sodium bicarbonate, it is desirable that any electrolyte component other than sodium bicarbonate is not contained, and the component substantially composed solely of sodium bicarbonate is preferred.

When the solid dialysis agent of the present invention is a three-pack type bicarbonate dialysis agent including a dialysis agent A-1, a dialysis agent A-2, and a dialysis agent B, the dialysis agent A-1 should be formulated by using the higher-order acetate compound and other electrolytes (other than the higher-order acetate compound and sodium bicarbonate). The dialysis agent A-2 of the three-pack type bicarbonate dialysis agent may be formulated by using glucose, it is desirable that any component other than glucose is not contained, and the component substantially composed solely of glucose is preferred. Also, the dialysis agent B of the three-pack type bicarbonate dialysis agent may be formulated by using sodium bicarbonate, it is desirable that any electrolyte component other than sodium bicarbonate is not contained, and the component substantially composed solely of sodium bicarbonate is preferred.

When the solid dialysis agent of the present invention is a three-pack type bicarbonate dialysis agent including a dialysis agent A, a dialysis agent S and a dialysis agent B, the solid agent A should be formulated by using the higher-order acetate compound, other electrolytes (other than the higher-order acetate compound and sodium bicarbonate), and, if necessary, glucose. The solid agent S of the three-pack type bicarbonate dialysis agent may be formulated by using sodium chloride, it is desirable that any electrolyte component other than sodium chloride is not contained, and the component substantially composed solely of sodium chloride is preferred. Also, the solid agent B of the three-pack type bicarbonate dialysis agent may be formulated by using sodium bicarbonate, it is desirable that any electrolyte component other than sodium bicarbonate is not contained, and the component substantially composed solely of sodium bicarbonate is preferred.

Among the types of bicarbonate dialysis agents, two-pack type bicarbonate dialysis agents including a dialysis agent A and a dialysis agent B and three-pack type dialysis agents including a dialysis agent A, a dialysis agent S and a dialysis agent B are preferable, from the viewpoint of ease of preparation of bicarbonate dialysate at clinical sites, preparation stability, and the like. In addition, preferred aspects of these two-pack type bicarbonate dialysis agents and three-pack type bicarbonate dialysis agents include an aspect in which glucose is contained in the dialysis agent A. As described above, since the higher-order acetate compound prevents decomposition of glucose and stabilizes glucose, preparation stabilization is achieved even when glucose is contained in the dialysis agent A.

In addition, in the case of the three-pack type bicarbonate dialysis agent including a dialysis agent A, a dialysis agent S and a dialysis agent B, as described in Japanese Patent No. 5099464, there is also an advantage that the sodium ion concentration and/or the bicarbonate ion concentration of the bicarbonate dialysate can be changed during dialysis treatment, according to pathology of patient.

Among the agents constituting each type of bicarbonate dialysis agent, an agent containing the higher-order acetate compound (that is, the one-pack type bicarbonate dialysis agent, the dialysis agent A agent, the dialysis agent A-1) should be in the form of a solid. Specific examples of the form of the agent containing the higher-order acetate compound include powder form, granule form, and the like.

The agents constituting each type of bicarbonate dialysis agent (one-pack type bicarbonate dialysis agent, dialysis agent A, dialysis agent A-1, dialysis agent A-2, dialysis agent S, dialysis agent B, and the like) can be prepared by selecting components to be blended according to the type of the agent, determining a blending amount of each component so that the composition and concentration of the bicarbonate dialysate finally prepared satisfy the above-mentioned ranges, and formulating according to the conventional formulation method of dialysis agent. Particularly, examples of a preferred method for producing a solid dialysis agent A having long-term storage stability include a method of obtaining a solid dialysis agent A through a step of granulating sodium chloride, calcium chloride, magnesium chloride and water to obtain electrolyte granules, and a step of mixing the electrolyte granules obtained in the above step, potassium chloride, the higher-order acetate compound, and glucose.

(Preparation of bicarbonate dialysate)

The bicarbonate dialysate is prepared by mixing the agents constituting the bicarbonate dialysis agent with a predetermined amount of water (preferably purified water) to dilute them.

EXAMPLES

Hereinafter, the present invention will be specifically described with reference to examples. However, the present invention is not construed as being limited to the following examples. The scope of the invention is defined in the claims.

Production Example: Production of higher-order acetate compound

Example 1

20.0 kg of glacial acetic acid, 24.6 kg of anhydrous sodium acetate and 18.3 kg of purified water were put in a filter dryer (manufacturer: TANABE WILLTEC INC, model number: TR25F) and stirred while heating until the product temperature reached 105°C to obtain a mixed solution in which glacial acetic acid and anhydrous sodium acetate were dissolved. About 9 kg of the mixed solution was evaporated and concentrated by continuing the stirring while maintaining the temperature, and then heating was stopped. Thereafter, cooling was carried out while continuing the stirring until the product temperature reached 42°C, and drying was carried out under reduced pressure at -96 to -98 kPa while stirring until the product temperature exceeded 60°C. The time of drying under reduced pressure was 150 minutes in total, the temperature of a jacket heating medium was kept at 50 to 55°C for 60 minutes from the start of drying under reduced pressure, and then the temperature of the jacket heating medium was controlled from 60°C to 80°C in stages for 90 minutes. The precipitation amount of crystals of the higher-order acetate compound increased with the lapse of time of drying under reduced pressure. Thereafter, the crystals were sieved with a sieve with an opening of 1.7 mm to obtain 37.2 kg of a higher-order acetate compound with good flowability.

Example 2

20.0 kg of glacial acetic acid, 24.6 kg of anhydrous sodium acetate and 18.3 kg of purified water were put in a filter dryer (manufacturer: TANABE WILLTEC INC, model number: TR25F) and stirred while heating until the product temperature reached 105°C to obtain a mixed solution in which glacial acetic acid and anhydrous sodium acetate were dissolved. About 9 kg of the mixed solution was evaporated and concentrated by continuing the stirring while maintaining the temperature, and then heating was stopped. Thereafter, cooling was carried out while continuing the stirring until the product temperature reached 43°C, and subsequently, drying was carried out under reduced pressure at -92 to -94 kPa while stirring until the product temperature exceeded 65°C. The time of drying under reduced pressure was 160 minutes in total, and the temperature of a jacket heating medium was kept at 90°C. The precipitation amount of crystals of the higher-order acetate compound increased with the lapse of time of drying under reduced pressure. Thereafter, the crystals were sieved with a sieve with an opening of 1.7 mm to obtain 27.0 kg of a higher-order acetate compound with good flowability.

Example 3

20.0 kg of glacial acetic acid, 24.6 kg of anhydrous sodium acetate and 18.3 kg of purified water were put in a filter dryer (manufacturer: TANABE WILLTEC INC, model number: TR25F) and stirred while heating until the product temperature reached 82°C to obtain a mixed solution in which glacial acetic acid and anhydrous sodium acetate were dissolved. Thereafter, an air vent valve at an upper part of a can body was opened, the temperature of a jacket heating medium was controlled to 80 to 85°C while ventilating indoor air to an inside of the can body, and drying was carried out under reduced pressure while maintaining the pressure at -50 to -52 kPa. The precipitation amount of crystals of the higher-order acetate compound increased with the lapse of time of drying under reduced pressure. When the product temperature reached 77°C by drying under reduced pressure, the pressure was returned to the atmospheric pressure, tap water was passed through the jacket, and when the product temperature reached 42°C, the crystals were sieved with a sieve with an opening of 1.7 mm to obtain 35.8 kg of a higher-order acetate compound with good flowability. The time of drying under reduced pressure was 330 minutes in total.

Example 4

20.0 kg of glacial acetic acid, 24.6 kg of anhydrous sodium acetate and 18.3 kg of purified water were put in a filter dryer (manufacturer: TANABE WILLTEC INC, model number: TR25F) and stirred while heating until the product temperature reached 90°C to obtain a mixed solution in which glacial acetic acid and anhydrous sodium acetate were dissolved. Thereafter, 1.5 kg of the higher-order acetate compound obtained in Example 2 was added. Next, an air vent valve at an upper part of a can body was opened, the temperature of a jacket heating medium was controlled to 80 to 90°C while ventilating indoor air to an inside of the can body, and drying was carried out under reduced pressure while controlling the pressure at -40 to -55 kPa in stages. The precipitation amount of crystals of the higher-order acetate compound increased with the lapse of time of drying under reduced pressure. When the product temperature exceeded 74°C by drying under reduced pressure, the pressure was returned to the atmospheric pressure, tap water was passed through the jacket, and when the product temperature reached 50°C, the crystals were sieved with a sieve with an opening of 1.7 mm to obtain 30.8 kg of a higher-order acetate compound with good flowability. The time of drying under reduced pressure was 195 minutes in total.

Example 5

20.0 kg of glacial acetic acid, 24.6 kg of anhydrous sodium acetate and 18.3 kg of purified water were put in a filter dryer (manufacturer: TANABE WILLTEC INC, model number: TR25F) and stirred while heating until the product temperature reached 78°C to obtain a mixed solution in which glacial acetic acid and anhydrous sodium acetate were dissolved. Thereafter, an air vent valve at an upper part of a can body was opened, the temperature of a jacket heating medium was controlled to 80 to 90°C while ventilating indoor air to an inside of the can body, and drying was carried out under reduced pressure while controlling the pressure at -30 to -90 kPa in stages. The precipitation amount of crystals of the higher-order acetate compound increased with the lapse of time of drying under reduced pressure. When the product temperature exceeded 75°C by drying under reduced pressure, the pressure was returned to the atmospheric pressure, tap water was passed through the jacket, and when the product temperature reached 45°C, the crystals were sieved with a sieve with an opening of 1.7 mm to obtain 41.9 kg of a higher-order acetate compound with good flowability. The time of drying under reduced pressure was 460 minutes in total.

Example 6

20.0 kg of glacial acetic acid, 24.6 kg of anhydrous sodium acetate and 18.3 kg of purified water were put in a filter dryer (manufacturer: TANABE WILLTEC INC, model number: TR25F) and stirred while heating until the product temperature reached 81°C to obtain a mixed solution in which glacial acetic acid and anhydrous sodium acetate were dissolved. Thereafter, an air vent valve at an upper part of a can body was opened, the temperature of a jacket heating medium was controlled to 80 to 90°C while ventilating indoor air to an inside of the can body, and drying was carried out under reduced pressure while controlling the pressure at -30 to -90 kPa in stages. The precipitation amount of crystals of the higher-order acetate compound increased with the lapse of time of drying under reduced pressure. When the product temperature exceeded 75°C by drying under reduced pressure, the pressure was returned to the atmospheric pressure, tap water was passed through the jacket, and when the product temperature reached 49°C, the crystals were sieved with a sieve with an opening of 1.7 mm to obtain 42.1 kg of an intermediate product of higher-order acetate compound with good flowability. The time of drying under reduced pressure was 640 minutes in total. About 100 g of this intermediate product of higher-order acetate compound was collected, placed on a metal pad, placed in an air dryer set at 80°C and heated for 450 minutes to obtain a higher-order acetate compound.

Example 7

About 100 g of the intermediate product of higher-order acetate compound obtained in the production process of Example 6 was collected, placed on a metal pad, placed in an air dryer set at 80°C and heated for 12 hours to obtain a higher-order acetate compound.

Example 8

About 100 g of the intermediate product of higher-order acetate compound obtained in the production process of Example 6 was collected, placed on a metal pad, placed in an air dryer set at 80°C and heated for 14 hours to obtain a higher-order acetate compound.

Example 9

About 100 g of the intermediate product of higher-order acetate compound obtained in the production process of Example 6 was collected, placed on a metal pad, placed in an air dryer set at 80°C and heated for 18 hours to obtain a higher-order acetate compound.

Example 10

30.0 kg of glacial acetic acid, 37.4 kg of anhydrous sodium acetate and 33.6 kg of purified water were put in a filter dryer (manufacturer: TANABE WILLTEC INC, model number: TR25F) with bubbling compressed air at 25 L/min, and stirred while heating until the product temperature reached 73°C to obtain a mixed solution in which glacial acetic acid and anhydrous sodium acetate were dissolved. Thereafter, the temperature of a jacket heating medium was kept at around 115°C while the valve at an upper part of a can body was closed, and drying was carried out under reduced pressure for 97 minutes while controlling the pressure from -75 to -86 kPa in stages. Next, when the product temperature exceeded 72°C, the temperature of the jacket heating medium was set to 85°C, and drying was carried out under reduced pressure for 18 minutes. The precipitation amount of crystals of the higher-order acetate compound increased with the lapse of time of drying under reduced pressure. Thereafter, the pressure was returned to the atmospheric pressure, tap water was passed through the jacket, and when the product temperature became 45°C or lower, the crystals were sieved with a sieve with an opening of 1.7 mm to obtain 53.0 kg of a higher-order acetate compound with good flowability. The time of drying under reduced pressure was 115 minutes in total.

Comparative Example 1

20.0 kg of glacial acetic acid, 24.6 kg of anhydrous sodium acetate and 18.3 kg of purified water were placed in a filter dryer (manufacturer: TANABE WILLTEC INC, model number: TR25F) and stirred while heating until the product temperature reached 100 to 105°C to obtain a mixed solution in which glacial acetic acid and anhydrous sodium acetate were dissolved. Subsequently, a part of purified water was volatilized and concentrated by continuing the stirring for 113 minutes while maintaining the temperature, and then heating was stopped. Thereafter, drying was carried out under reduced pressure while stirring at -95 to -97 kPa for 80 minutes. From the start of drying under reduced pressure, the temperature of the jacket heating medium was controlled to 57 to 60°C. Meanwhile, the product temperature rose gradually from 42 to 46°C, and the precipitation amount of crystals of the higher-order acetate compound increased with the lapse of time. Thereafter, the crystals were sieved with a sieve with an opening of 1.7 mm to obtain a higher-order acetate compound with good flowability.

Comparative Example 2

42.3 g of glacial acetic acid and 57.8 g of anhydrous sodium acetate were placed in a plastic bag and thoroughly mixed to obtain a higher-order acetate compound.

Test Example 1: Evaluation of physical properties of higher-order acetate compounds

(1) Powder X-ray diffraction

Measurement was carried out in the range of 2Θ = 5 to 90° by an X-ray diffractometer "SmartLab" (manufacturer: Rigaku Corporation) (measurement conditions were as follows; target: Cu, tube voltage: 40 kV, tube current: 30 mA, scanning range: 5 to 90°, scan speed: 10.000°/min, scan step: 0.02°, scan mode: continuous). The measurement results were analyzed using Rigaku Data Analysis Software PDXL version 2.0.3.0 to obtain integrated intensities of each peak.

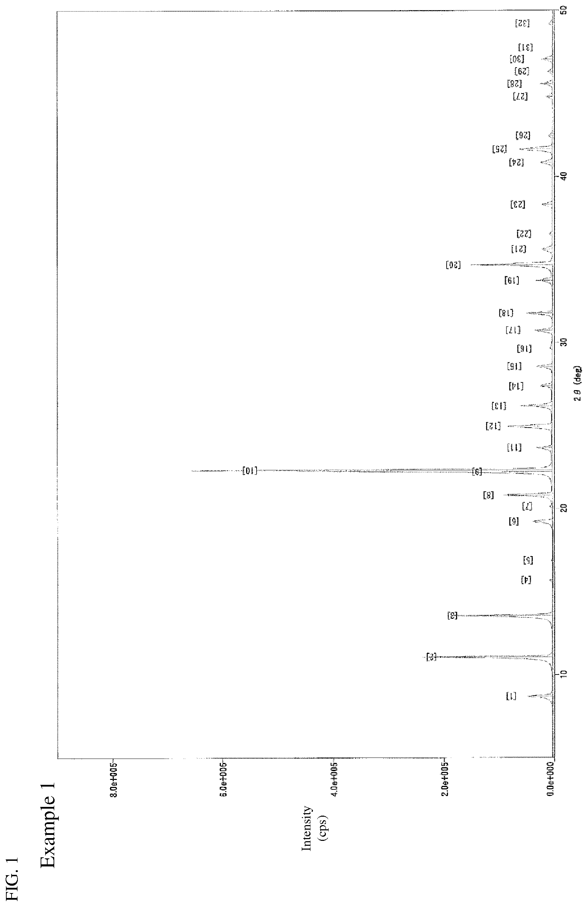

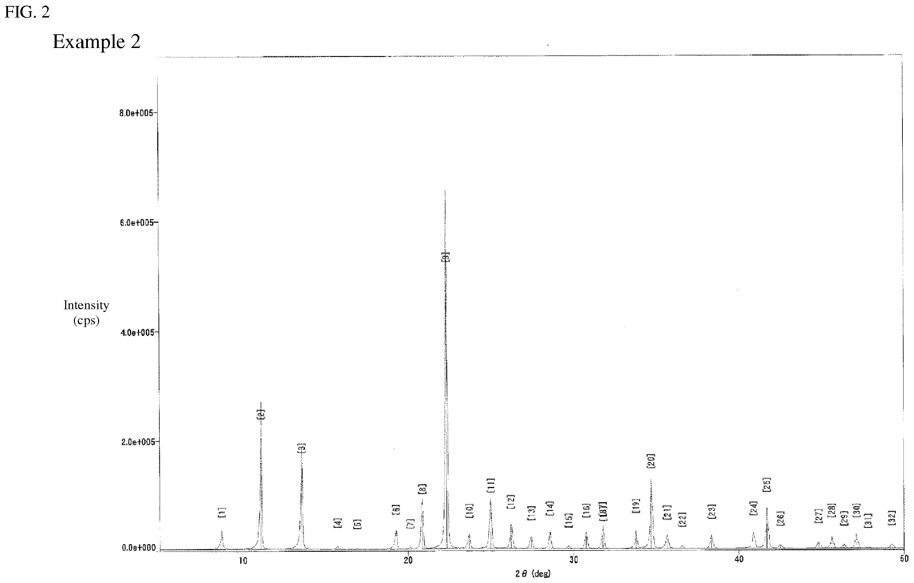

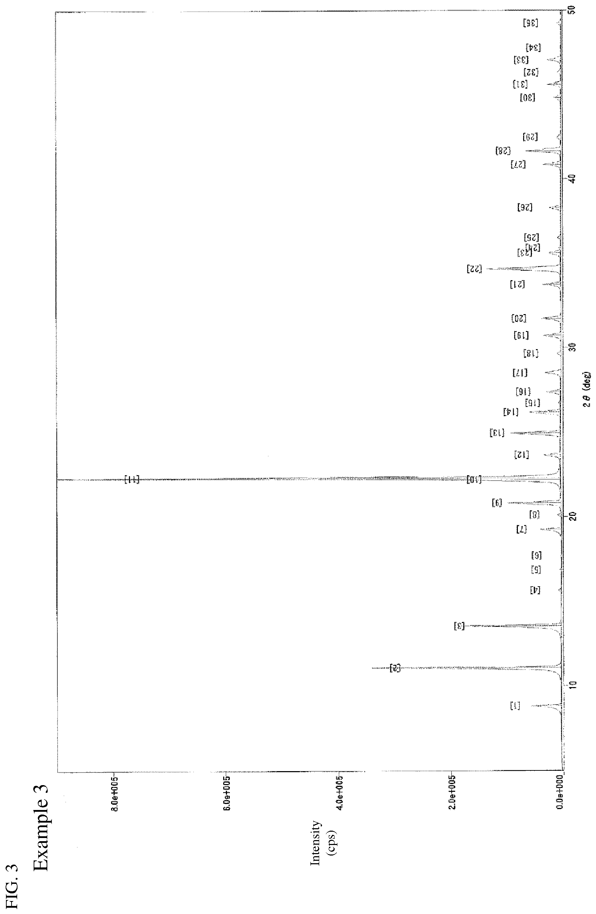

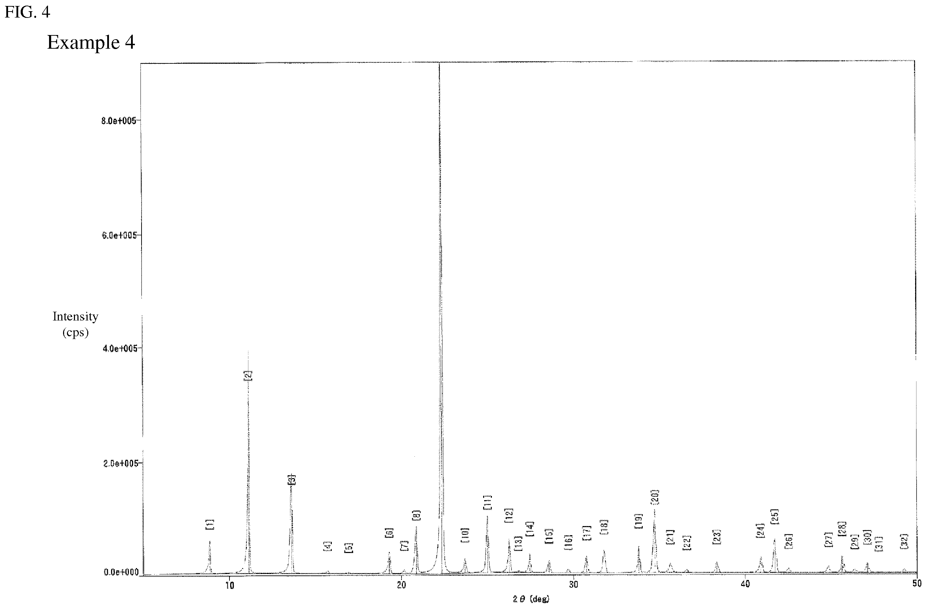

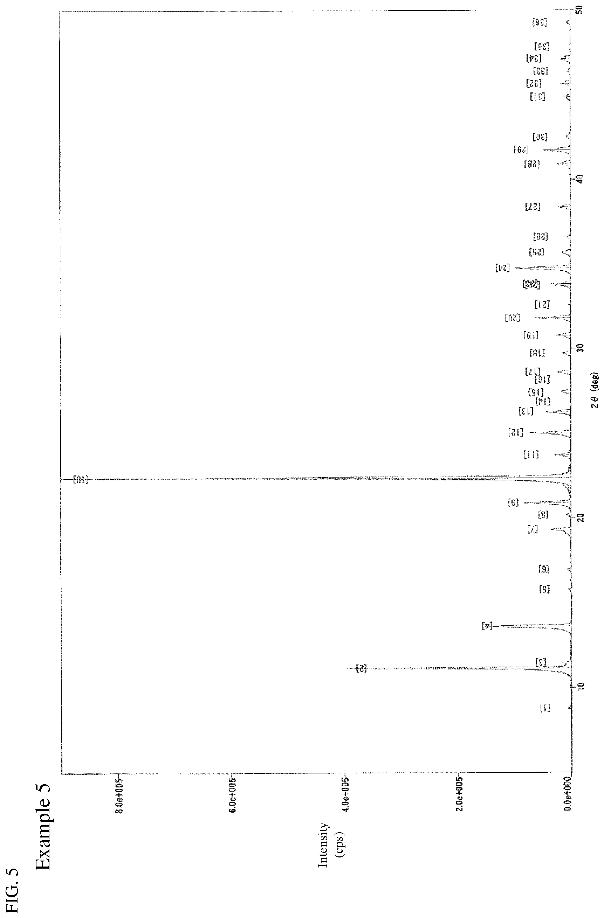

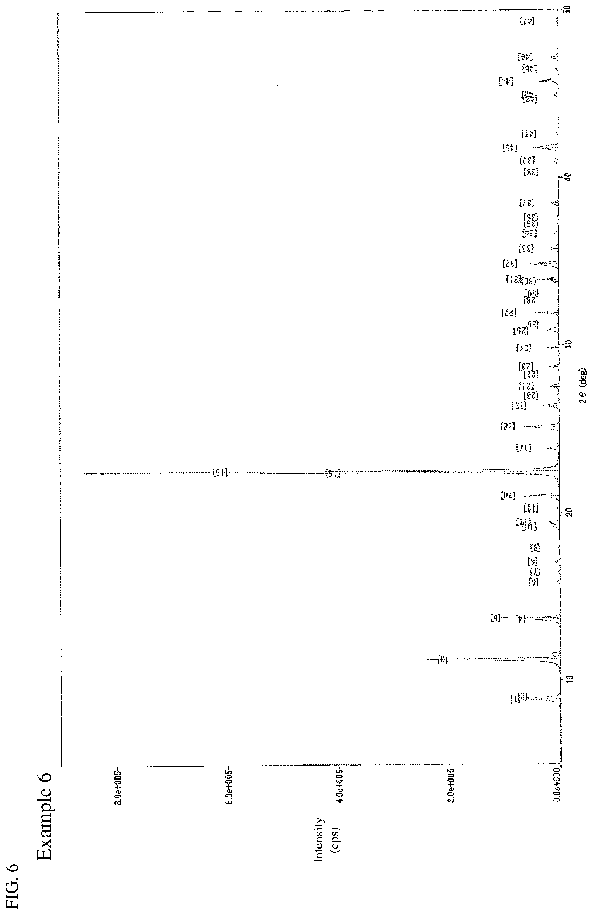

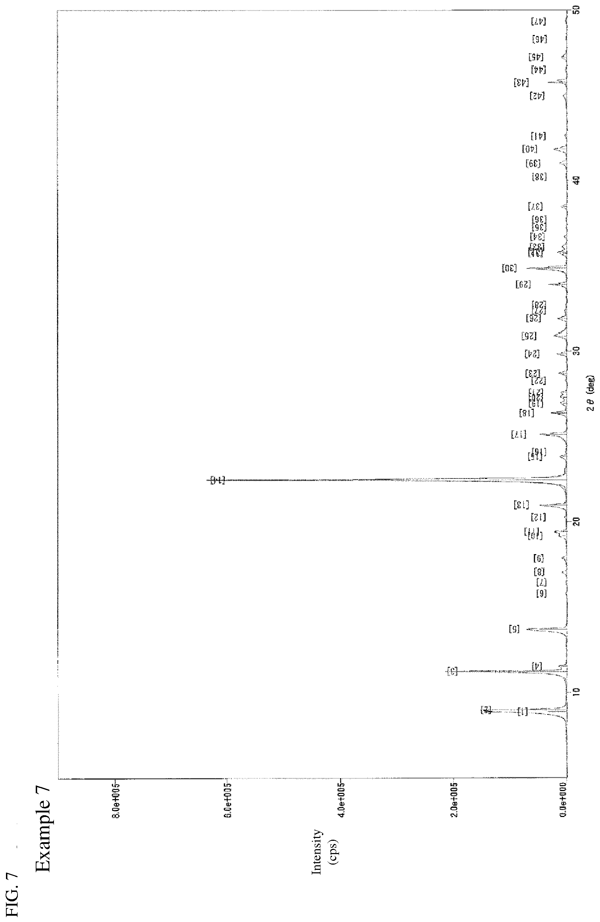

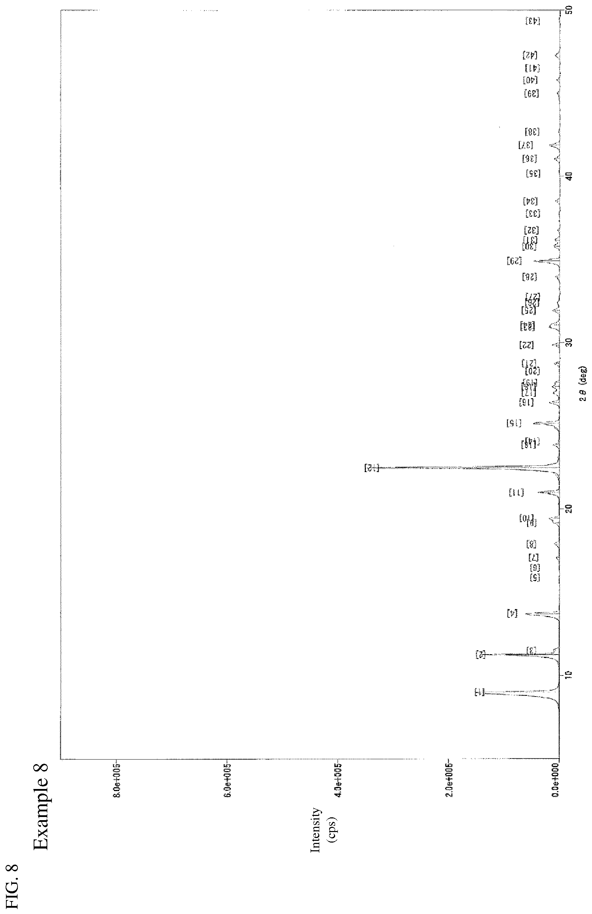

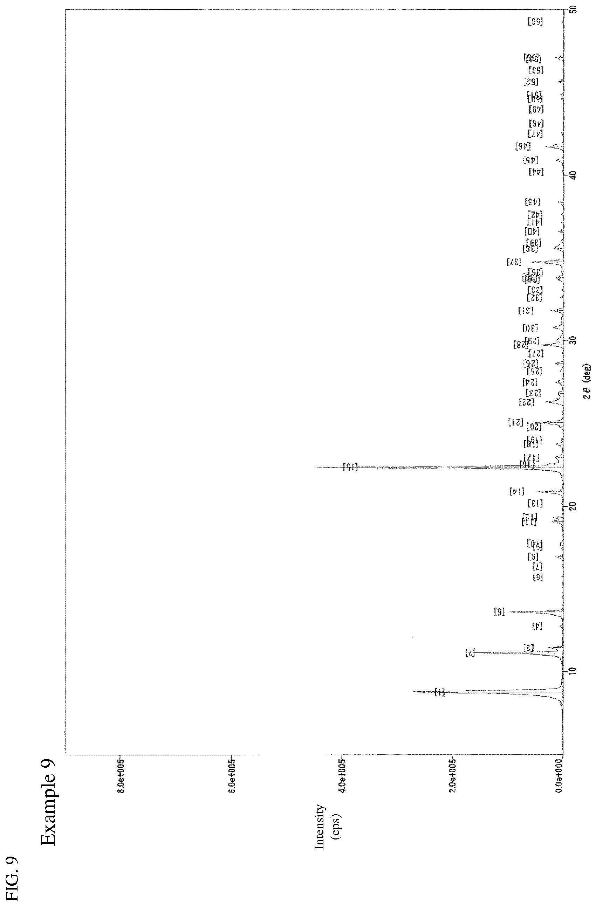

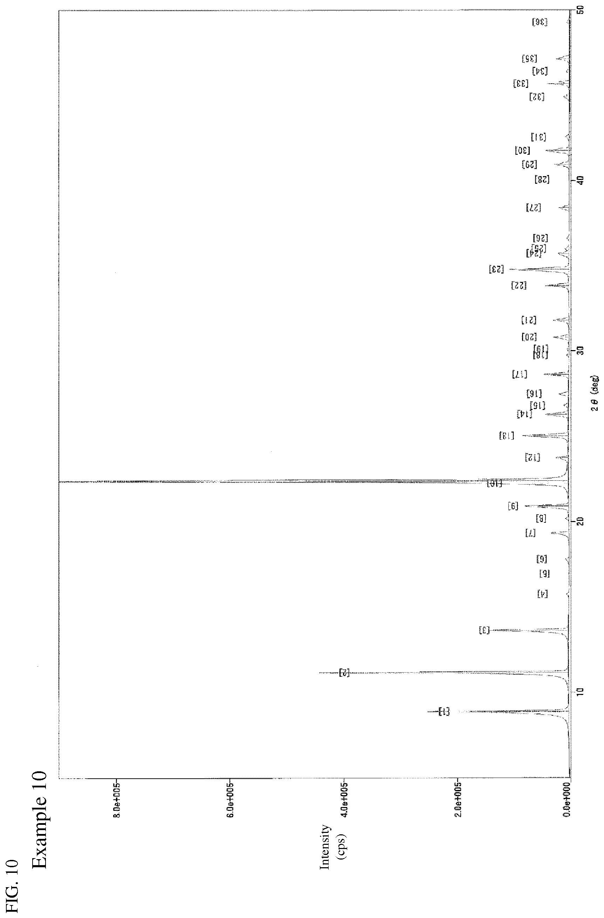

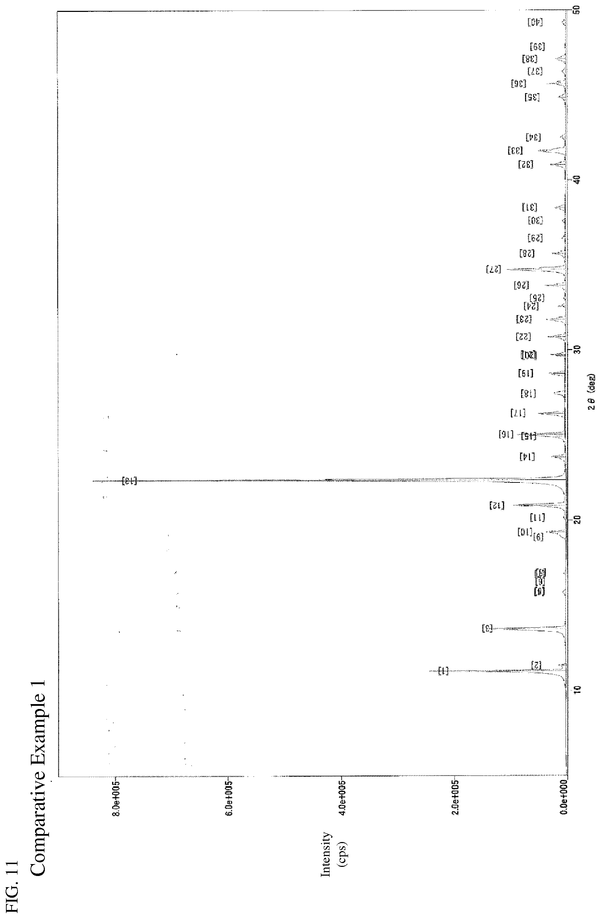

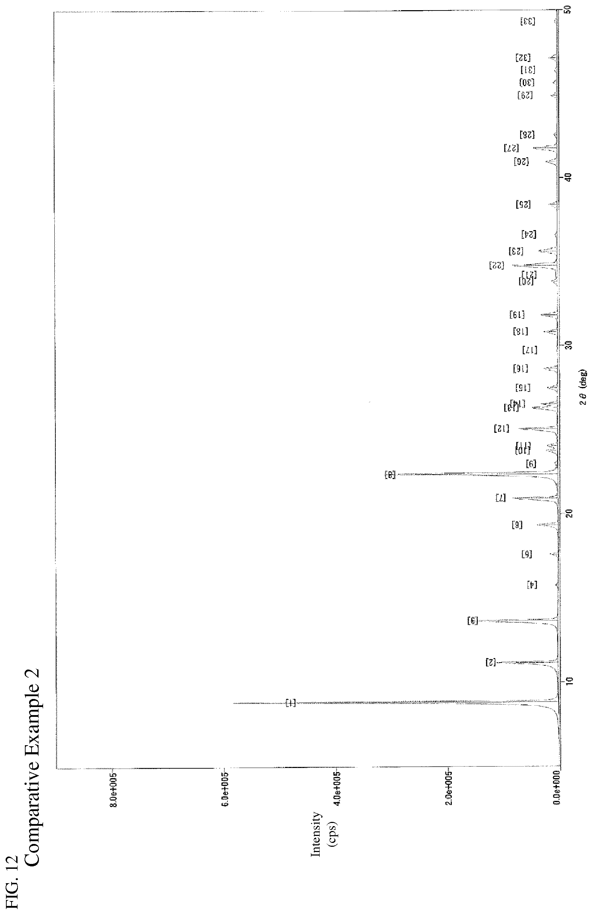

Figs. 1 to 12 show powder X-ray diffraction patterns of the higher-order acetate compounds of Examples 1 to 10 and Comparative Examples 1 and 2, respectively. Also, Table 2 shows results of determining an integrated intensity (Ia) of diffraction peak at 2θ = 8.8° ± 0.2°, an integrated intensity (Ib) of diffraction peak at 2Θ = 22.3° ± 0.2°, an integrated intensity (Ic) of diffraction peak at 2Θ = 17.0° ± 0.2°, and a ratio Ia/Ib and a ratio Ia/Ic of each higher-order acetate compound. In addition, Table 3 shows peaks observed by powder X-ray diffraction of each higher-order acetate compound and integrated intensities of each peak. As a result, in the higher-order acetate compounds of Examples 1 to 10 which were sufficiently dried under reduced pressure on a mixed solution of glacial acetic acid, anhydrous sodium acetate and water, the diffraction peak at 2Θ = 8.8° ± 0.2° was observed, and the ratio Ia/Ib was 1.138 or less. On the other hand, in the higher-order acetate compound of Comparative Example 1 which was dried under reduced pressure for a short time on a mixed solution of glacial acetic acid, anhydrous sodium acetate and water, no diffraction peak at 2Θ = 8.8° ± 0.2° was observed. Furthermore, the higher-order acetate compound of Comparative Example 2 obtained by mixing glacial acetic acid and anhydrous sodium acetate had a small integrated intensity of diffraction peak at 2Θ = 22.3° ± 0.2° as compared with the cases of Examples 1 to 10 and Comparative Example 1, and the ratio Ia/Ib was as high as 1.447.

[Table 2]

|  | Integrated intensity (Ia) of diffraction peak at 2θ = 8.8° ± 0.2° | Integrated intensity (Ib) of diffraction peak at 2θ = 22.3° ± 0.2° | Integrated intensity (Ic) of diffraction peak at 2θ = 17.0° ± 0.2° | Ratio Ia/Ib | Ratio Ia/Ic |
|---|---|---|---|---|---|
| Example 1 | 6208 (8.72°) | 57863 (22.33°) | 357 (16.90°) | 0.107 | 17.389 |
| Example 2 | 4726 (8.75°) | 69161 (22.35°) | 285 (16.93°) | 0.068 | 16.582 |
| Example 3 | 6584 (8.83°) | 60153 (22.33°) | 360 (16.89°) | 0.109 | 18.289 |
| Example 4 | 6082 (8.87°) | 99867 (22.36°) | 259 (16.93°) | 0.061 | 23.483 |
| Example 5 | 749 (8.80°) | 86652 (22.39°) | 642 (16.99°) | 0.009 | 1.167 |
| Example 6 | 7760 (8.87°) | 44943 (22.49°) | 1030 (17.06°) | 0.173 | 7.534 |
| Example 7 | 19008 (8.97°) | 60110 (22.47°) | 991 (17.06°) | 0.316 | 19.181 |
| Example 8 | 30615 (9.00°) | 40464 (22.48°) | 727 (17.09°) | 0.757 | 42.111 |
| Example 9 | 44161 (8.78°) | 38805 (22.39°) | 1548 (16.98°) | 1.138 | 28.528 |
| Example 10 | 28703 (8.88°) | 87411 (22.40°) | 85 (16.96°) | 0.328 | 337.682 |

(continued)

| | Integrated intensity (Ia) of diffraction peak at 2θ = 8.8° ± 0.2° | Integrated intensity (Ib) of diffraction peak at 2θ = 22.3° ± 0.2° | Integrated intensity (Ic) of diffraction peak at 2θ = 17.0° ± 0.2° | Ratio Ia/Ib | Ratio Ia/Ic |
|---|---|---|---|---|---|
| Comparative Example 1 | 0 | 75270 (22.39°) | 1570 (19.03°) | 0 | 0 |
| Comparative Example 2 | 55191 (8.83°) | 38144 (22.38°) | 0 | 1.447 | - |

[Table 3]

| No | Example 1 | | Example 2 | | Example 3 | | Example 4 | | Example 5 | | Example 6 | | Example 7 | | Example 8 | | Example 9 | | Example 10 | | Comparative Example 1 | | Comparative Example 2 | |
|---|---|---|---|---|---|---|---|---|---|---|---|---|---|---|---|---|---|---|---|---|---|---|---|---|
| | 2θ | Integrated intensity | 2θ | Integrated intensity | 2θ | Integrated intensity | 2θ | Integrated intensity | 2θ | Integrated intensity | 2θ | Integrated intensity | 2θ | Integrated intensity | 2θ | Integrated intensity | 2θ | Integrated intensity | 2θ | Integrated intensity | 2θ | Integrated intensity | 2θ | Integrated intensity |
| 1 | 8.72 | 6208 | 8.75 | 4726 | 8.83 | 6584 | 8.87 | 6082 | 8.80 | 749 | 8.87 | 7760 | 8.87 | 9374 | 9.00 | 30615 | 8.78 | 44161 | 8.88 | 28703 | 11.16 | 21652 | 8.83 | 55191 |
| 2 | 11.09 | 22361 | 11.11 | 23340 | 11.09 | 30010 | 11.14 | 31134 | 11.17 | 32306 | 8.99 | 3149 | 8.97 | 19008 | 11.26 | 14665 | 11.15 | 14965 | 11.17 | 35824 | 11.49 | 1579 | 11.18 | 15000 |
| 3 | 13.57 | 19694 | 13.59 | 20029 | 13.58 | 18750 | 13.64 | 20641 | 11.47 | 2254 | 11.25 | 25180 | 11.24 | 17481 | 11.50 | 2267 | 11.46 | 3850 | 13.64 | 16215 | 13.65 | 17778 | 13.69 | 20070 |
| 4 | 15.72 | 733 | 15.74 | 653 | 15.69 | 489 | 15.73 | 570 | 13.66 | 19073 | 13.64 | 5084 | 11.52 | 3818 | 13.75 | 9954 | 12.76 | 581 | 15.77 | 530 | 15.79 | 409 | 15.79 | 638 |
| 5 | 16.90 | 357 | 16.93 | 285 | 16.89 | 360 | 16.93 | 259 | 15.80 | 414 | 13.72 | 7220 | 13.73 | 10746 | 15.88 | 197 | 13.65 | 10595 | 16.96 | 85 | 15.84 | 330 | 17.62 | 1523 |
| 6 | 19.24 | 4924 | 19.28 | 4878 | 17.74 | 257 | 19.30 | 5276 | 16.99 | 642 | 15.86 | 473 | 15.81 | 235 | 16.47 | 177 | 15.73 | 213 | 17.81 | 992 | 16.40 | 68 | 19.35 | 5153 |
| 7 | 20.14 | 353 | 20.15 | 304 | 19.27 | 4928 | 20.18 | 833 | 19.35 | 5775 | 16.44 | 227 | 16.44 | 203 | 17.09 | 727 | 16.39 | 344 | 19.34 | 3894 | 16.85 | 358 | 20.95 | 10629 |
| 8 | 20.81 | 10319 | 20.87 | 10725 | 20.14 | 556 | 20.89 | 10494 | 20.21 | 817 | 17.06 | 1030 | 17.06 | 991 | 17.89 | 1889 | 16.98 | 1548 | 20.20 | 441 | 16.93 | 5 | 22.38 | 38144 |
| 9 | 22.23 | 12330 | 22.35 | 69161 | 20.85 | 10689 | 22.36 | 99867 | 20.92 | 10060 | 17.89 | 427 | 17.86 | 1594 | 19.18 | 2444 | 17.60 | 249 | 20.92 | 8735 | 19.03 | 1570 | 22.98 | 589 |
| 10 | 22.33 | 57863 | 23.71 | 3441 | 22.22 | 29546 | 23.71 | 3934 | 22.39 | 86652 | 19.15 | 1623 | 19.18 | 3062 | 19.45 | 2322 | 17.77 | 1067 | 22.26 | 25473 | 19.32 | 4109 | 23.78 | 2387 |
| 11 | 23.68 | 3599 | 25.02 | 10967 | 22.33 | 60153 | 25.03 | 12018 | 23.75 | 3587 | 19.44 | 2933 | 19.43 | 2191 | 21.00 | 5434 | 19.07 | 2239 | 22.40 | 87411 | 20.15 | 1007 | 24.06 | 2551 |
| 12 | 24.99 | 10081 | 26.20 | 5896 | 23.69 | 3149 | 26.26 | 6775 | 25.06 | 8726 | 20.23 | 309 | 20.28 | 427 | 22.48 | 40464 | 19.35 | 2525 | 23.76 | 2717 | 20.88 | 10964 | 25.09 | 9216 |
| 13 | 26.20 | 7062 | 26.20 | 2659 | 24.99 | 10255 | 26.79 | 228 | 26.30 | 5075 | 20.30 | 158 | 21.00 | 5911 | 23.87 | 1815 | 20.20 | 293 | 25.03 | 9744 | 22.39 | 75270 | 26.33 | 6155 |
| 14 | 27.37 | 2600 | 27.44 | 2659 | 26.23 | 6350 | 27.47 | 3547 | 26.88 | 153 | 21.01 | 6961 | 22.47 | 60110 | 24.07 | 310 | 20.91 | 5497 | 26.30 | 5107 | 23.73 | 3875 | 26.56 | 2389 |
| 15 | 28.56 | 3336 | 28.58 | 3864 | 26.75 | 364 | 28.58 | 2789 | 27.43 | 2243 | 22.41 | 36809 | 23.82 | 1839 | 25.16 | 7657 | 22.39 | 38805 | 26.80 | 1026 | 24.95 | 3892 | 27.52 | 2370 |
| 16 | 29.63 | 923 | 29.71 | 569 | 27.42 | 2831 | 29.72 | 764 | 28.17 | 150 | 22.49 | 44943 | 24.10 | 301 | 26.41 | 2648 | 22.55 | 1543 | 27.50 | 2112 | 25.05 | 6955 | 28.66 | 2998 |
| 17 | 30.74 | 3884 | 30.76 | 3863 | 28.53 | 3464 | 30.78 | 3706 | 28.64 | 2917 | 23.83 | 2429 | 25.13 | 7420 | 27.00 | 2204 | 22.95 | 3315 | 28.62 | 4619 | 26.30 | 5524 | 29.75 | 387 |
| 18 | 31.78 | 5730 | 31.77 | 4131 | 29.67 | 763 | 31.80 | 4955 | 29.75 | 1809 | 25.15 | 8021 | 26.36 | 3642 | 27.37 | 2205 | 23.75 | 1610 | 29.76 | 456 | 27.45 | 2446 | 30.84 | 3048 |
| 19 | 33.77 | 3816 | 31.81 | 793 | 30.74 | 3819 | 33.82 | 4404 | 30.78 | 3321 | 26.36 | 3424 | 26.92 | 1725 | 27.61 | 1326 | 24.02 | 458 | 30.11 | 466 | 28.63 | 3313 | 31.87 | 4266 |
| 20 | 34.71 | 17616 | 34.72 | 16233 | 31.77 | 4236 | 34.77 | 14460 | 31.84 | 6063 | 26.99 | 593 | 27.29 | 2172 | 28.30 | 121 | 24.80 | 1158 | 30.81 | 3612 | 29.67 | 997 | 33.85 | 1291 |
| 21 | 35.64 | 3214 | 35.67 | 3539 | 33.77 | 3570 | 35.65 | 2802 | 32.63 | 219 | 27.51 | 2094 | 27.56 | 1183 | 28.75 | 1329 | 25.06 | 5665 | 31.83 | 3766 | 29.74 | 1155 | 34.25 | 2177 |
| 22 | 36.56 | 593 | 36.58 | 605 | 34.71 | 16504 | 36.60 | 642 | 33.77 | 2011 | 28.24 | 304 | 28.26 | 217 | 29.86 | 1646 | 26.27 | 5316 | 33.85 | 4117 | 30.78 | 3789 | 34.81 | 10549 |
| 23 | 38.32 | 2446 | 38.36 | 2927 | 35.65 | 3064 | 38.35 | 2362 | 33.84 | 2340 | 28.72 | 1866 | 28.68 | 1776 | 30.94 | 2087 | 26.83 | 1497 | 34.80 | 13431 | 31.84 | 4349 | 35.64 | 5196 |
| 24 | 40.86 | 3146 | 40.89 | 4024 | 35.95 | 351 | 40.89 | 3649 | 34.79 | 12768 | 29.82 | 2648 | 29.84 | 1939 | 31.06 | 3125 | 27.48 | 2211 | 35.73 | 2874 | 32.60 | 903 | 36.65 | 560 |
| 25 | 41.67 | 8540 | 41.69 | 9345 | 36.55 | 647 | 41.72 | 8304 | 35.71 | 2429 | 30.84 | 2900 | 30.88 | 5513 | 31.97 | 1741 | 28.15 | 443 | 36.02 | 1036 | 33.08 | 234 | 38.42 | 2133 |
| 26 | 42.49 | 868 | 42.50 | 1114 | 38.33 | 2417 | 42.53 | 1061 | 36.62 | 709 | 31.19 | 508 | 31.93 | 2065 | 32.43 | 1037 | 28.61 | 1783 | 36.62 | 482 | 33.84 | 39990 | 40.96 | 3101 |
| 27 | 44.81 | 1701 | 44.83 | 1870 | 40.85 | 3725 | 44.85 | 1720 | 38.39 | 2747 | 31.93 | 4468 | 32.40 | 715 | 32.75 | 193 | 29.24 | 119 | 38.40 | 2186 | 34.79 | 13691 | 41.77 | 6319 |
| 28 | 45.58 | 2602 | 45.61 | 2729 | 41.67 | 8229 | 45.62 | 2809 | 40.91 | 3400 | 32.70 | 335 | 32.71 | 191 | 33.96 | 1138 | 29.75 | 3815 | 40.05 | 277 | 35.69 | 3194 | 42.55 | 1014 |
| 29 | 46.34 | 1059 | 46.35 | 966 | 42.48 | 1134 | 46.36 | 872 | 41.75 | 6683 | 33.12 | 137 | 33.93 | 3386 | 34.92 | 6884 | 29.98 | 3162 | 40.94 | 3502 | 36.60 | 633 | 44.91 | 1472 |
| 30 | 47.07 | 2804 | 47.11 | 3585 | 44.80 | 1987 | 47.10 | 2377 | 42.51 | 974 | 33.80 | 1584 | 34.88 | 9214 | 35.79 | 1906 | 30.75 | 2760 | 41.75 | 5988 | 37.62 | 565 | 45.67 | 1017 |
| 31 | 47.76 | 158 | 47.80 | 190 | 45.59 | 2635 | 47.76 | 163 | 44.86 | 1734 | 33.93 | 1984 | 35.75 | 1898 | 36.17 | 1514 | 31.83 | 2733 | 42.53 | 1057 | 38.39 | 2415 | 46.42 | 622 |
| 32 | 49.23 | 953 | 49.25 | 1096 | 46.31 | 792 | 49.25 | 932 | 45.66 | 1938 | 34.87 | 7712 | 35.82 | 468 | 36.76 | 526 | 32.61 | 389 | 44.88 | 1456 | 40.91 | 3772 | 47.15 | 2357 |
| 33 | - | - | - | - | 47.04 | 3238 | - | - | 46.39 | 610 | 35.74 | 2775 | 36.11 | 1463 | 37.76 | 339 | 33.08 | 209 | 45.67 | 3702 | 41.73 | 6949 | 49.32 | 724 |
| 34 | - | - | - | - | 47.76 | 154 | - | - | 47.13 | 2762 | 36.67 | 784 | 36.70 | 582 | 38.50 | 1093 | 33.70 | 943 | 46.39 | 688 | 42.51 | 1290 | - | - |
| 35 | - | - | - | - | 49.23 | 873 | - | - | 47.84 | 138 | 37.25 | 241 | 37.26 | 156 | 40.17 | 550 | 33.83 | 1033 | 47.13 | 3186 | 44.86 | 1839 | - | - |
| 36 | - | - | - | - | - | - | - | - | 49.28 | 888 | 37.61 | 338 | 37.71 | 228 | 41.07 | 1477 | 34.14 | 1625 | 49.29 | 769 | 45.65 | 3348 | - | - |
| 37 | - | - | - | - | - | - | - | - | - | - | 38.47 | 1716 | 38.48 | 1300 | 41.89 | 3184 | 34.78 | 6105 | - | - | 46.38 | 893 | - | - |
| 38 | - | - | - | - | - | - | - | - | - | - | 40.31 | 197 | 40.23 | 290 | 42.68 | 408 | 35.57 | 2805 | - | - | 47.12 | 2601 | - | - |

| Row | | | | | |
|-----|------|------|------|------|------|
| 39 | 41.01 1961 | 40.99 2244 | 44.97 1505 | 35.94 1741 | 47.84 137 |
| 40 | 41.76 6802 | 41.84 3792 | 45.78 837 | 36.62 950 | 49.28 872 |
| 41 | 42.60 773 | 42.60 611 | 46.50 296 | 37.16 324 | |
| 42 | 44.68 341 | 44.96 1738 | 47.27 1799 | 37.61 410 | |
| 43 | 44.93 1027 | 45.74 3132 | 49.33 286 | 38.39 1444 | |
| 44 | 45.73 3801 | 46.47 367 | | 40.22 455 | |
| 45 | 46.46 | 47.22 1952 | | 40.92 2185 | |
| 46 | 47.19 | 48.27 180 | | 41.74 4665 | |
| 47 | 49.35 | 49.33 458 | | 42.52 506 | |
| 48 | | | | 43.12 150 | |
| 49 | | | | 43.99 169 | |
| 50 | | | | 44.60 388 | |
| 51 | | | | 44.86 888 | |
| 52 | | | | 45.65 1206 | |
| 53 | | | | 46.36 281 | |
| 54 | | | | 47.01 746 | |
| 55 | | | | 47.12 1340 | |
| 56 | | | | 49.28 558 | |

(2) Median diameter

The median diameters of the higher-order acetate compounds of Examples 1 to 10 and Comparative Examples 1 and 2 were measured by using sieves with openings of 850 $\mu$m, 710 $\mu$m, 500 $\mu$m, 355 $\mu$m, 250 $\mu$m, 180 $\mu$m, 150 $\mu$m and 106 $\mu$m, using a robot shifter (manufacturer: SEISHIN ENTERPRISE Co., Ltd., model number: RPS-105), under conditions of a sound wave intensity of 20, a sound wave frequency of 51 Hz, a classification time of 5 min, sweep time of 0.3 min, and pulse interval of 1 sec. Table 4 shows the results of the median diameter obtained from the measurement results.

[Table 4]

|  | Median diameter ($\mu$m) |
|---|---|
| Example 1 | 593.9 |
| Example 2 | 486.7 |
| Example 3 | 427.7 |
| Example 4 | 287.7 |
| Example 5 | 644.7 |
| Example 6 | 674.2 |
| Example 7 | 689.5 |
| Example 8 | 742.9 |
| Example 9 | 702.7 |
| Example 10 | 374.0 |
| Comparative Example 1 | 637.8 |
| Comparative Example 2 | 600.5 |

(3) Observation of crystal form with scanning electron microscope

The crystal form of each obtained higher-order acetate compound was observed with a scanning electron microscope (manufacturer: JEOL Ltd., model number: JSM-5500LV).

Fig. 13 shows the result of observing the higher-order acetate compound of Example 2 with a scanning electron microscope. As a result, it was confirmed that the higher-order acetate compound of Example 2 was mainly approximately cubic crystal.

Test Example 2: Stability evaluation of dialysis agent A containing higher-order acetate compound

(1) Production of electrolyte granules

First, 40.2 kg of sodium chloride, 1.339 kg of calcium chloride hydrate and 0.911 kg of magnesium chloride hydrate were heated and mixed, further purified water was added thereto, and the mixture was mixed (manufacturer: Hosokawa Micron Corporation, model number: NX-2J) and then dried with a fluidized bed dryer (manufacturer: NAGATO ELECTRIC co., ltd., model number: 10F) at 150°C for 10 minutes. The dried product was sieved with a sieve with an opening of 1.7 mm to obtain electrolyte granules.

(2) Preparation of dialysis agent A

In an environment of low temperature and low humidity (15°C, 15% RH), 115.7 g of the electrolyte granules, 3.00 g of potassium chloride, 26.25 g of glucose, and 5.27 g of each of the higher-order acetate compounds of Examples 1 to 10 and Comparative Examples 1 and 2 were mixed in a plastic bag so that the components are uniformly mixed, and the mixture was stored in a laminated bag (moisture permeability was substantially 0 g/m$^2$ · 24 h) formed by a laminate in which a polyethylene terephthalate film, an aluminum foil and a polyethylene film were laminated from an inner layer toward an outer layer and sealed by heat sealing to obtain a dialysis agent A.

(3) Method for evaluating stability of dialysis agent A

Each of the dialysis agents A containing each higher-order acetate compound was subjected to a storage test at 50°C for 15 days. In addition to the storage test at 50°C, the dialysis agent A containing the higher-order acetate compound obtained in Example 1 was subjected to a storage test at 40°C for 2 months, and the dialysis agent A containing the higher-order acetate compound obtained in Example 10 was also subjected to a storage test at 55°C for 15 days. Volatile acetic acid concentration, pH, 5-HMF, solidification degree and coloration were measured by the following methods, for each dialysis agent A before storage, after 5 days of storage, after 10 days of storage and after 15 days of storage in the storage test at 50°C, for the dialysis agent A after 2 months in the storage test at 40°C, and for the dialysis agent A after 5 days of storage and after 15 days of storage in the storage test at 55°C.

<Volatile acetic acid concentration>

The laminated bag storing the dialysis agent A containing each higher-order acetate compound was opened, and an acetic acid detection tube was set in a detector tube type gas measuring instrument (manufacturer: GASTEC Corporation, model number: GV-100S), and the volatile acetic acid concentration was measured by ventilating a certain amount of sample gas.

<5-Hydroxymethylfurfural (5-HMF)>

The laminated bag containing the dialysis agent A containing each higher-order acetate compound was opened, and the whole contents were dissolved in purified water to make the total volume to 500 mL and filtered with a filter with a pore size of 0.2 $\mu$m to obtain a 35-fold concentrated agent A solution of dialysate. With respect to the obtained 35-fold concentrated agent A solution, an absorbance at a wavelength of 284 nm was measured using a spectrophotometer, thereby measuring an amount of 5-hydroxymethylfurfural (hereinafter described as 5-HMF) in the 35-fold concentrated agent A solution. 5-HMF is a compound generated by decomposition of glucose, and the lower the absorbance, the more stably the glucose is maintained.

<pH>

pH of the 35-fold concentrated agent A solution used for measuring the 5-HMF amount was measured at a liquid temperature of 25°C using a pH meter (manufacturer: HORIBA, Ltd., model number: F-73).

<Presence or absence of solidification and coloration>

After measuring the volatile acetic acid concentration, the presence or absence of solidification of the preparation in the bag was confirmed by hand touch from outside the bag. Further, the preparation in the bag was visually observed, and the presence or absence of coloration was confirmed. A normal dialysis agent A without glucose degradation is white, and when degradation progresses, it turns yellow or brown.

(4) Evaluation result

Tables 5 to 10 show the obtained results. As a result, in the dialysis agents A containing the higher-order acetate compounds of Examples 1 to 10, the volatile acetic acid concentration was small, and decomposition of glucose could be sufficiently prevented even after 15 days of storage at 50°C, and further, no pH change, solidification and coloration were observed, and preparation stability was excellent. In addition, degradation of glucose, pH change, solidification, and coloration were not observed even after two months of storage at 40°C, and long-term storage stability was excellent. On the other hand, in the dialysis agent A containing the higher-order acetate compound of Comparative Example 1, despite having a molar ratio of acetic acid to sodium acetate nearly equivalent to those of Examples 1 to 5, after 15 days of storage at 50°C, the volatile acetic acid concentration was remarkably high, remarkable decomposition of glucose was also observed, and further, the preparation was in a solidified state. In addition, in the dialysis agent A containing the higher-order acetate compound of Comparative Example 2, despite having a molar ratio of acetic acid to sodium acetate nearly equivalent to those of Examples 1 to 5, the volatile acetic acid concentration was high from the storage start, and after 5 days or later of storage at 50°C, remarkable decomposition of glucose was also observed, and further, solidification and coloration of the preparation occurred.

From the above results, in the higher-order acetate compounds containing acetic acid-sodium acetate mixed crystals, when peak A at 2Θ = 8.8° ± 0.2° and peak B at 2Θ = 22.3° ± 0.2° were observed, and a ratio Ia/Ib of integrated intensity Ia of the peak A to integrated intensity Ib of the peak B is less than 1.447, it was revealed that acetic acid odor can be reduced, and decomposition of glucose can be prevented even when coexisted with glucose. Further, it was also revealed that the higher-order acetate compound having the above characteristics can improve preparation stability when it is contained in the dialysis agent A.

[Table 5]

| At start | | | | | |
|---|---|---|---|---|---|
| Higher-order acetate compound used | Volatile acetic acid concentration (ppm) | 5-HMF (Abs) | pH | Solidification (present or absent) | Coloration (present or absent) |
| Example 1 | 2 | 0.0000 | 4.31 | Absent | Absent |
| Example 2 | 2 | 0.0002 | 4.32 | Absent | Absent |
| Example 3 | 5 | 0.0001 | 4.31 | Absent | Absent |
| Example 4 | 8 | 0.0002 | 4.32 | Absent | Absent |
| Example 5 | 1 | 0.0017 | 4.30 | Absent | Absent |
| Example 6 | 1 | 0.0014 | 4.41 | Absent | Absent |
| Example 7 | 1 | 0.0015 | 4.49 | Absent | Absent |
| Example 8 | 0 | 0.0018 | 4.55 | Absent | Absent |
| Example 9 | 1 | 0.0025 | 4.63 | Absent | Absent |
| Example 10 | 0 | 0.0030 | 4.33 | Absent | Absent |
| Comparative Example 1 | 3 | 0.0012 | 4.31 | Absent | Absent |
| Comparative Example 2 | 900 | 0.0001 | 4.30 | Absent | Absent |

[Table 6]

| After 5 days of storage at 50°C | | | | | |
|---|---|---|---|---|---|
| Higher-order acetate compound used | Volatile acetic acid concentration (ppm) | 5-HMF (Abs) | pH | Solidification (present or absent) | Coloration (present or absent) |
| Example 1 | 10 | 0.0013 | 4.31 | Absent | Absent |
| Example 2 | 10 | 0.0011 | 4.33 | Absent | Absent |
| Example 3 | 10 | 0.0013 | 4.32 | Absent | Absent |
| Example 4 | 10 | 0.0016 | 4.32 | Absent | Absent |
| Example 5 | 5 | 0.0020 | 4.35 | Absent | Absent |
| Example 6 | 5 | 0.0018 | 4.47 | Absent | Absent |
| Example 7 | 3 | 0.0018 | 4.54 | Absent | Absent |
| Example 8 | 5 | 0.0019 | 4.58 | Absent | Absent |
| Example 9 | 3 | 0.0019 | 4.69 | Absent | Absent |
| Example 10 | 10 | 0.0018 | 4.32 | Absent | Absent |
| Comparative Example 1 | 5 | 0.0006 | 4.32 | Absent | Absent |
| Comparative Example 2 | >1000 | 1.2642 | 4.36 | Present | Present |

[Table 7]

| After 10 days of storage at 50°C | | | | | |
|---|---|---|---|---|---|
| Higher-order acetate compound used | Volatile acetic acid concentration (ppm) | 5-HMF (Abs) | pH | Solidification (present or absent) | Coloration (present or absent) |
| Example 1 | 20 | 0.0017 | 4.31 | Absent | Absent |
| Example 2 | 15 | 0.0017 | 4.32 | Absent | Absent |
| Example 3 | 30 | 0.0016 | 4.31 | Absent | Absent |
| Example 4 | 25 | 0.0017 | 4.31 | Absent | Absent |
| Example 5 | 20 | 0.0016 | 4.34 | Absent | Absent |
| Example 6 | 10 | 0.0017 | 4.46 | Absent | Absent |
| Example 7 | 5 | 0.0016 | 4.55 | Absent | Absent |
| Example 8 | 5 | 0.0017 | 4.60 | Absent | Absent |
| Example 9 | 2 | 0.0015 | 4.69 | Absent | Absent |
| Example 10 | 10 | 0.0022 | 4.32 | Absent | Absent |
| Comparative Example 1 | 30 | 0.0024 | 4.31 | Absent | Absent |
| Comparative Example 2 | >1000 | 3.8782 | 4.35 | Present | Present |

[Table 8]

| After 15 days of storage at 50°C | | | | | |
|---|---|---|---|---|---|
| Higher-order acetate compound used | Volatile acetic acid concentration (ppm) | 5-HMF (Abs) | pH | Solidification (present or absent) | Coloration (present or absent) |
| Example 1 | 10 | 0.0020 | 4.31 | Absent | Absent |
| Example 2 | 7 | 0.0021 | 4.30 | Absent | Absent |
| Example 3 | 25 | 0.0021 | 4.31 | Absent | Absent |
| Example 4 | 70 | 0.0018 | 4.30 | Absent | Absent |
| Example 5 | 35 | 0.0025 | 4.30 | Absent | Absent |
| Example 6 | 5 | 0.0020 | 4.41 | Absent | Absent |
| Example 7 | 10 | 0.0020 | 4.49 | Absent | Absent |
| Example 8 | 7 | 0.0020 | 4.55 | Absent | Absent |
| Example 9 | 10 | 0.0018 | 4.62 | Absent | Absent |
| Example 10 | 50 | 0.0039 | 4.34 | Absent | Absent |
| Comparative Example 1 | >1000 | 0.1873 | 4.38 | Present | Absent |
| Comparative Example 2 | >1000 | 3.8920 | 4.41 | Present | Present |

[Table 9]

| Result of storage test at 40°C of Example 1 | | | | | |
|---|---|---|---|---|---|
| Period | Volatile acetic acid concentration (ppm) | 5-HMF (Abs) | pH | Solidification (present or absent) | Coloration (present or absent) |
| At start | 2 | 0.0000 | 4.31 | Absent | Absent |

(continued)

| Result of storage test at 40°C of Example 1 | | | | | |
| --- | --- | --- | --- | --- | --- |
| Period | Volatile acetic acid concentration (ppm) | 5-HMF (Abs) | pH | Solidification (present or absent) | Coloration (present or absent) |
| After 2 months of storage | 10 | 0.0015 | 4.31 | Absent | Absent |

[Table 10]

| Result of storage test at 55°C of Example 10 | | | | | |
| --- | --- | --- | --- | --- | --- |
| Period | Volatile acetic acid concentration (ppm) | 5-HMF (Abs) | pH | Solidification (present or absent) | Coloration (present or absent) |
| At start | 0 | 0.0030 | 4.33 | Absent | Absent |
| After 5 days of storage | 10 | 0.0018 | 4.32 | Absent | Absent |
| After 15 days of storage | 90 | 0.0047 | 4.36 | Absent | Absent |

**Claims**

1. A higher-order acetate compound comprising acetic acid-sodium acetate mixed crystals,

   wherein a diffraction peak A at $2\Theta = 8.8° \pm 0.2°$ and a diffraction peak B at $2\Theta = 22.3° \pm 0.2°$ are observed in powder X-ray diffraction measurement, and
   a ratio $I_a/I_b$ of integrated intensity $I_a$ of the diffraction peak A to integrated intensity $I_b$ of the diffraction peak B is 0.001 to 1.140.

2. A solid dialysis agent A, wherein the solid dialysis agent A comprises the higher-order acetate compound according to claim 1 and sodium chloride or
   the solid dialysis agent A comprises the higher-order acetate compound according to claim 1 and is substantially free of sodium chloride.

3. The solid dialysis agent A according to claim 2, further comprising glucose.

4. A solid dialysis agent comprising the higher-order acetate compound according to claim 1.

5. A solid dialysis agent according to claim 4, wherein the solid dialysis agent is a two-pack type bicarbonate dialysis agent comprising:

   the solid dialysis agent A according to claim 2 or 3; which comprises sodium chloride and
   a dialysis agent B containing sodium bicarbonate.

6. A solid dialysis agent according to claim 4, wherein the solid dialysis agent is a three-pack type bicarbonate dialysis agent comprising:

   the solid dialysis agent A according to claim 2 or 3, which is substantially free of sodium chloride;
   a dialysis agent S containing sodium chloride; and
   a dialysis agent B containing sodium bicarbonate.

7. A method for producing the higher-order acetate compound comprising acetic acid-sodium acetate mixed crystals according to claim 1, the method comprising:

a step 1 of mixing acetic acid, sodium acetate and an aqueous solvent to obtain a mixed solution; and
a step 2 of drying the mixed solution obtained in the step 1 under reduced pressure, until a higher-order acetate compound is produced, in which the diffraction peak A at $2\Theta = 8.8° \pm 0.2°$ and a diffraction peak B at $2\Theta = 22.3° \pm 0.2°$ are observed and a ratio Ia/Ib of integrated intensity Ia of the diffraction peak A to integrated intensity of Ib of the diffraction peak B is from 0.001 to 1.140.

8. The production method according to claim 7, wherein the drying under reduced pressure in the step 2 is performed under a pressure condition of -30 to -100 kPa.

**Patentansprüche**

1. Acetatverbindung höherer Ordnung, die Essigsäure-Natriumacetat-Mischkristalle umfasst,

   wobei bei einer Röntgenbeugungsmessung ein Beugungsreflex A bei $2\theta = 8,8° \pm 0,2°$ und ein Beugungsreflex B bei $2\theta = 22,3 ° \pm 0,2°$ erkennbar sind und
   das Verhältnis Ia/Ib zwischen der integrierten Intensität Ia des Beugungsreflexes A und der integrierten Intensität Ib des Beugungsreflexes B 0,001 bis 1,140 beträgt.

2. Festes Dialysemittel A, wobei das feste Dialysemittel A eine Acetatverbindung höherer Ordnung nach Anspruch 1 und Natriumchlorid umfasst oder
   das feste Dialysemittel A eine Acetatverbindung höherer Ordnung nach Anspruch 1 umfasst und im Wesentlichen frei von Natriumchlorid ist.

3. Festes Dialysemittel A nach Anspruch 2, das weiters Glucose umfasst.

4. Festes Dialysemittel, das eine Acetatverbindung höherer Ordnung nach Anspruch 1 umfasst.

5. Festes Dialysemittel nach Anspruch 4, wobei das feste Dialysemittel ein Bicarbonat-Dialysemittel vom Zweipackungstyp ist, das Folgendes umfasst:

   ein festes Dialysemittel A nach Anspruch 2 oder 3, das Natriumchlorid umfasst, und
   ein Dialysemittel B, das Natriumbicarbonat umfasst.

6. Festes Dialysemittel nach Anspruch 4, wobei das feste Dialysemittel ein Bicarbonat-Dialysemittel vom Dreipackungstyp ist, das Folgendes umfasst:

   ein festes Dialysemittel A nach Anspruch 2 oder 3, das im Wesentlichen frei von Natriumchlorid ist,
   ein Dialysemittel S, das Natriumchlorid umfasst, und
   ein Dialysemittel B, das Natriumbicarbonat umfasst.

7. Verfahren zur Herstellung einer Acetatverbindung höherer Ordnung, die Essigsäure-Natriumacetat-Mischkristalle umfasst, nach Anspruch 1, wobei das Verfahren Folgendes umfasst:

   einen Schritt 1 des Vermischens von Essigsäure, Natriumacetat und einem wässrigen Lösungsmittel, um eine gemischte Lösung zu erhalten; und
   einen Schritt 2 des Trocknens der in Schritt 1 erhaltenen gemischten Lösung unter reduziertem Druck, bis eine Acetatverbindung höherer Ordnung erhalten wird, wobei ein Beugungsreflex A bei $2\theta = 8,8° \pm 0,2°$ und ein Beugungsreflex B bei $2\theta = 22,3 ° \pm 0,2°$ erkennbar sind und das Verhältnis Ia/Ib zwischen der integrierten Intensität Ia des Beugungsreflexes A und der integrierten Intensität Ib des Beugungsreflexes B 0,001 bis 1,140 beträgt.

8. Herstellungsverfahren nach Anspruch 7, wobei das Trocknen unter reduziertem Druck in Schritt 2 unter der Druckbedingung von -30 bis -100 kPa erfolgt.

**Revendications**

1. Composé d'acétate d'ordre supérieur comprenant des cristaux mixtes d'acide acétique-acétate de sodium,

   dans lequel un pic de diffraction A à 2θ = 8,8° ± 0,2° et un pic de diffraction B à 2θ = 22,3° ± 0,2° sont observés lors d'une mesure de diffraction des rayons X sur poudre, et
   un rapport Ia/Ib entre l'intensité intégrée Ia du pic de diffraction A et l'intensité intégrée Ib du pic de diffraction B est de 0,001 à 1,140.

2. Agent de dialyse solide A, dans lequel l'agent de dialyse solide A comprend le composé d'acétate d'ordre supérieur selon la revendication 1 et du chlorure de sodium ou
   l'agent de dialyse solide A comprend le composé d'acétate d'ordre supérieur selon la revendication 1 et est sensiblement exempt de chlorure de sodium.

3. Agent de dialyse solide A selon la revendication 2, comprenant en outre du glucose.

4. Agent de dialyse solide comprenant le composé d'acétate d'ordre supérieur selon la revendication 1.

5. Agent de dialyse solide selon la revendication 4, dans lequel l'agent de dialyse solide est un agent de dialyse bicarbonate de type à deux composants comprenant :

   l'agent de dialyse solide A selon la revendication 2 ou 3 ; qui comprend du chlorure de sodium et
   un agent de dialyse B contenant du bicarbonate de sodium.

6. Agent de dialyse solide selon la revendication 4, dans lequel l'agent de dialyse solide est un agent de dialyse bicarbonate de type à trois composants comprenant :

   l'agent de dialyse solide A selon la revendication 2 ou 3, qui est sensiblement exempt de chlorure de sodium ;
   un agent de dialyse S contenant du chlorure de sodium ; et
   un agent de dialyse B contenant du bicarbonate de sodium.

7. Procédé de production du composé d'acétate d'ordre supérieur comprenant des cristaux mixtes d'acide acétique-acétate de sodium selon la revendication 1, le procédé comprenant :

   une étape 1 de mélange d'acide acétique, d'acétate de sodium et d'un solvant aqueux pour obtenir une solution mixte ; et
   une étape 2 de séchage de la solution mixte obtenue à l'étape 1 sous pression réduite, jusqu'à production d'un composé d'acétate d'ordre supérieur, dans laquelle le pic de diffraction A à 2θ = 8,8° ± 0,2° et un pic de diffraction B à 2θ = 22,3° ± 0,2° sont observés et un rapport Ia/Ib entre l'intensité intégrée Ia du pic de diffraction A et l'intensité intégrée Ib du pic de diffraction B est de 0,001 à 1,140.

8. Procédé de production selon la revendication 7, dans lequel le séchage sous pression réduite à l'étape 2 est réalisé sous une condition de pression de -30 à -100 kPa.

FIG. 1

Example 1

FIG. 2

Example 2

FIG. 3

Example 3

FIG. 4

Example 4

Intensity
(cps)

FIG. 5

Example 5

FIG. 6

Example 6

FIG. 7

Example 7

FIG. 8

Example 8

EP 3 533 777 B1

FIG. 9

Example 9

Intensity (cps)

2 θ (deg)

FIG. 10

Example 10

FIG. 11

Comparative Example 1

FIG. 12
Comparative Example 2

FIG. 13

(SEM Image of Example 2)

FIG. 14

Anhydrous sodium acetate

$2\theta$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5099464 B
- JP 5517321 B
- JP H759846 A
- WO 201572494 A
- CN 104892397 A

**Non-patent literature cited in the description**

- General Tests, Processes and Apparatus, 3.04 Particle Size Determination, 2. Method 2. Analytical Sieving Method. The Japanese Pharmacopoeia